# EUROPEAN PATENT APPLICATION

(11) **EP 4 585 594 A1**
(43) Date of publication of application: **16.07.2025**
(21) Application number: 23862941.4
(22) Date of filing: 24.08.2023
(51) Int. Cl.: C07D 405/14, C07D 491/18, C08G 59/02, C08G 59/40, C08L 63/00

(54) **GLYCIDYL GROUP-CONTAINING COMPOUND, CURABLE RESIN COMPOSITION, CURED PRODUCT AND MULTILAYER BODY**

(30) Priority: 06.09.2022 JP 2022141291
(71) Applicant: DIC Corporation, Tokyo 174-8520 (JP)
(72) Inventor: ARITA Kazuo, Sakura-shi, Chiba 285-8668 (JP); SUZUKI Etsuko, Sakura-shi, Chiba 285-8668 (JP)
(74) Representative: Kraus & Lederer PartGmbB
(86) International application number: PCT/JP2023/030419
(87) International publication number: WO 2024/053402

(57) **Abstract**

An object is to provide a compound which is advantageous in that a cured article obtained from the compound, despite being a curable resin, can easily achieve healing ability, remoldability, and dismantlability, a curable resin composition using the compound and a cured article obtained therefrom. Specifically, there is used a glycidyl group-containing compound comprising a conjugated diene structure (A) having at least one glycidyl group and a dienophile structure (B) having at least one glycidyl group, in which the conjugated diene structure (A) and the dienophile structure (B) are linked together by the Diels-Alder reaction. It is preferred that the conjugated diene structure (A) is a furan-derived structure or an anthracene-derived structure, and the dienophile structure (B) is a maleimide-derived structure.

## Description

### TECHNICAL FIELD

The present invention relates to a glycidyl group-containing compound having a specific structure, a curable resin composition containing the compound, a cured article, and a laminated product comprising a layer formed from the cured article.

### BACKGROUND ART

A cured article obtained from an epoxy resin has excellent heat resistance, mechanical strength, electric properties, adhesion properties, and the like, and is an indispensable material in various fields, such as the electric or electronic field, and the field of coating composition and adhesive.

Meanwhile, with respect to the cured article using a thermosetting resin, such as an epoxy resin, a disadvantage is pointed out such that the long-term reliability is low, and, for example, it is likely that when a cured article of an epoxy resin suffers deterioration by oxidation, cracks are formed in the cured article.

Further, the cured article obtained by curing a thermosetting resin, such as an epoxy resin, cannot be dissolved in a solvent (insoluble), and further is not dissolved even at high temperatures (infusible). It is difficult to recycle or reuse such a cured article, and the used cured article remains as waste. Therefore, the cured article must achieve a reduction of the waste and burden on the environment.

For this reason, the cured articles using an epoxy resin and the like are required to solve problems about an increase of the life of the products and a reduction of waste, and, for solving the problems, it is considered that imparting dismantlability, healing ability, and remoldability to the cured article is effective.

Under the circumstances, a method is disclosed in which a heat decomposable compound is preliminarily blended into a reactive adhesive component, and, after being used, the adhesive is subjected to predetermined heating to lower the bonding strength, so that the adhesive can be dismantled (see, for example, PTL 1).

In addition, a method is disclosed in which, with respect to an encapsulation material using an epoxy resin or the like, by using a first thermosetting resin and microcapsule particles containing therein a second thermosetting resin precursor substance in the encapsulation material, the resultant encapsulation material has self-heating ability even when a crack is formed or peeling is caused (see, for example, PTL 2).

Further, as a method for imparting self-heating ability to an epoxy resin cured article, incorporation of a bonding site by the Diels-Alder reaction is provided (see, for example, NPL 1).

In addition to the above-mentioned methods, studies are frequently being made on using reversible bonding, such as dynamic covalent bonding or supermolecular bonding, in the cured article for imparting healing ability and remoldability.

### CITATION LIST

### PATENT LITERATURE

PTL 1: JP2013-256557A
PTL 2: JP2017-041496A

### NON PATENT LITERATURE

NPL 1: Amendola, E., Iacono, S.D., Pastore, A., Curcio, M., Giordano, M. and Iadonisi, A. "Epoxy Thermosets with Self-Healing Ability", Journal of Materials Science and Chemical Engineering, 2015, 3, p162-167

### SUMMARY OF INVENTION

### TECHNICAL PROBLEM

In the technique provided in PTL 1, the dismantled adhesive is disposed of, and the substrate which is a material to be adhered is removed can be recycled, but there is a drawback in that the collective recycling properties are unsatisfactory. Further, in the technique of PTL 2, the method has certain self-heating ability, but is not a means for solving the problem in view of reuse, and problems of the unnecessary waste remain. In the technique provided in NPL 1, there is a possibility that the low-molecular compound formed due to the Retro-Diels-Alder reaction volatilizes from the cured article to cause a weight loss or contamination of the mold used for remolding.

Further, the raw materials used in connection with the reversible bonding pose a problem in that, due to a need for securing the molecular motion, the raw material is limited to the use of a substance in a gel form, which has poor mechanical strength, and thus any of the prior art methods are required to be improved. Accordingly, a task of the invention is to provide a compound which is advantageous in that a cured article obtained from the compound, despite being a curable resin, can easily achieve healing ability, remoldability, and dismantlability, and a curable resin composition using the compound and a cured article obtained therefrom.

### SOLUTION TO PROBLEM

The present inventors have conducted extensive and intensive studies. As a result, it has been found that the above-mentioned problems can be solved by using a glycidyl group-containing compound having a specific structure in the form of a curable resin composition, and the invention has been completed.

Specifically, the invention includes the following embodiments.
[1] A glycidyl group-containing compound including a conjugated diene structure (A) having at least one glycidyl group and a dienophile structure (B) having at least one glycidyl group, in which the conjugated diene structure (A) and the dienophile structure (B) are linked together by the Diels-Alder reaction.
[2] The glycidyl group-containing compound according to item [1] above, in which the dissociation temperature of the linked portion by the Diels-Alder reaction is 100°C or higher.
[3] The glycidyl group-containing compound according to item [1] or [2] above, in which the conjugated diene structure (A) is a furan-derived structure or an anthracene-derived structure.
[4] The glycidyl group-containing compound according to any one of items [1] to [3] above, in which the dienophile structure (B) is a maleimide-derived structure.
[5] A glycidyl group-containing compound which is represented by the following general formula: 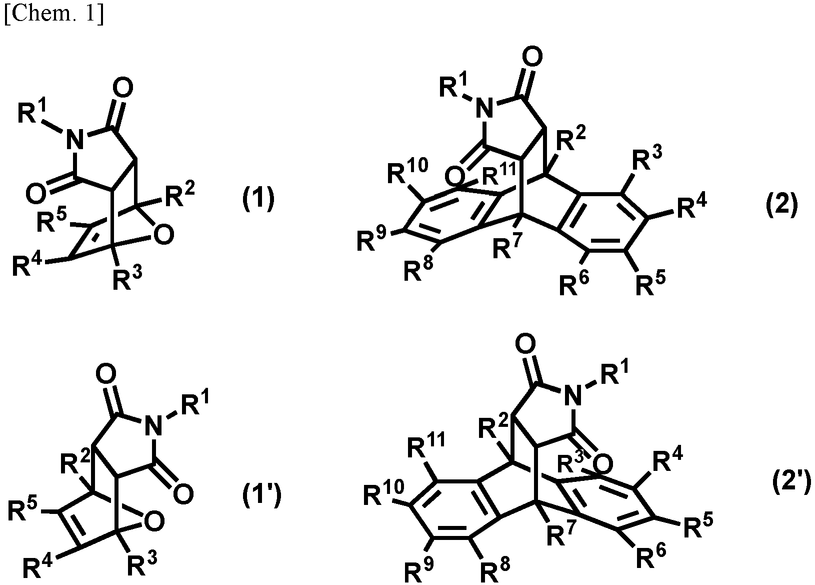 in the formula, R¹ is a glycidyl group or a substituent having at least one glycidyl group, and each of R² to R¹¹ is independently a hydrogen atom, a halogen atom, a hydroxyl group, a nitro group, a carboxyl group, a cyano group, an alkoxy group, an aralkyloxy group, an aryloxy group, an amino group, an amide group, an alkyloxycarbonyl group, an aryloxycarbonyl group, an alkyl group, a cycloalkyl group, an aralkyl group, an aryl group, or a glycidyl group,
   and may have a substituent on the carbon atom or nitrogen atom of the alkoxy group, aralkyloxy group, aryloxy group, amino group, amide group, alkyloxycarbonyl group, aryloxycarbonyl group, alkyl group, cycloalkyl group, aralkyl group, aryl group, or glycidyl group,
   with the proviso that at least one of R² to R⁵ in the formula (1) or (1') or at least one of R² to R¹¹ in the formula (2) or (2') is a glycidyl group or a group having at least one glycidyl group.
[6] The glycidyl group-containing compound according to any one of items [1] to [5] above, which has an epoxy equivalent in the range of 100 to 500 g/eq.
[7] A curable resin composition containing the glycidyl group-containing compound according to any one of items [1] to [6] above, and a compound (I) having reactivity with the glycidyl group-containing compound as essential components.
[8] The curable resin composition according to item [7] above, in which the compound (I) having reactivity with the glycidyl group-containing compound is a hydroxyl group-containing compound.
[9] The curable resin composition according to item [7] or [8] above, further containing an epoxy resin which is other than the glycidyl group-containing compound according to any one of items [1] to [5] above, and which has an epoxy equivalent of 100 to 10,000 g/eq.
[10] The curable resin composition according to item [9] above, in which the epoxy resin is represented by the following formula (3) and has an epoxy equivalent of 500 to 10,0000 g/eq: 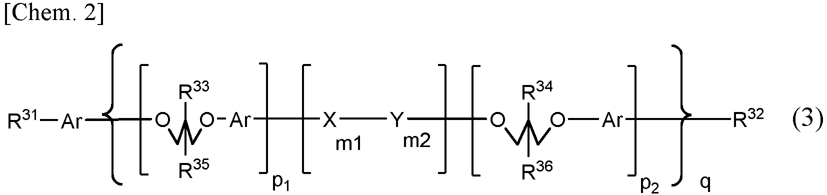 in the formula (3), each Ar is independently a structure having an aromatic ring which is unsubstituted or which has a substituent,
   X is a structural unit represented by the following formula (3-1), Y is a structural unit represented by the following formula (3-2): 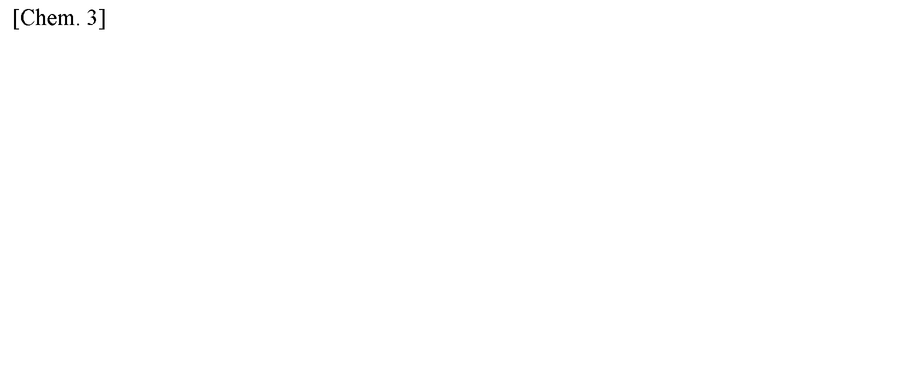 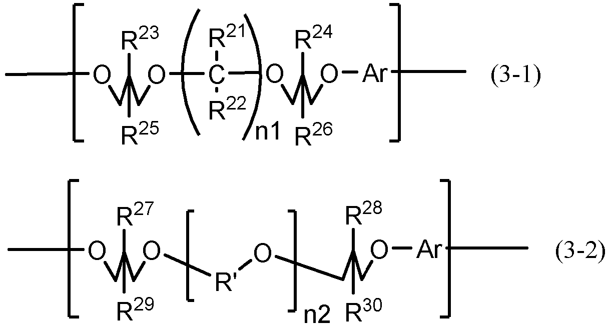 in the formulae (3-1) and (3-2), Ar is as defined above,
      each of R²¹ and R²² is independently a hydrogen atom, a methyl group, or an ethyl group, R' is a divalent hydrocarbon group having 2 to 12 carbon atoms,
      each of R²³, R²⁴, R²⁷, and R²⁸ is independently a hydroxyl group, a glycidyl ether group, or a 2-methylglycidyl ether group,
      each of R²⁵, R²⁶, R²⁹, and R³⁰ is independently a hydrogen atom or a methyl group,
      n₁ is an integer of 4 to 16, and
      n₂ represents an average of the numbers of the repeating units and is 2 to 30,
   each of R³¹ and R³² is independently a glycidyl ether group or a 2-methylglycidyl ether group,
   each of R³³ and R³⁴ is independently a hydroxyl group, a glycidyl ether group, or a 2-methylglycidyl ether group,
   R³⁵ and R³⁶ are a hydrogen atom or a methyl group, and
   m1, m2, p1, p2, and q are an average of the numbers of repetition,
   in which each of m1 and m2 is independently 0 to 25, and m1 and m2 satisfy the relationship: m1 + m2 ≥ 1,
   each of p1 and p2 is independently 0 to 5, and
   q is 0.5 to 5,
   with the proviso that the bonding of the structural unit X represented by the formula (3-1) above and the structural unit Y represented by the formula (3-2) above may be in a random or block form, and the total number of structural units X and the total number of structural units Y present per molecule are m1 and m2, respectively.
[11] The curable resin composition according to any one of items [7] to [10] above, in which the concentration of the Diels-Alder reaction unit is 0.10 mmol/g or more, based on the total mass of the curable components of the curable resin composition.
[12] The curable resin composition according to any one of items [7] to [11] above, which is a self-heating composition, a composition for a remolding material, or a dismantlable composition.
[13] A cured article which is obtained by curing the curable resin composition according to any one of items [7] to [12] above.
[14] A laminated product having a substrate, and a layer including the cured article according to item [13] above.
[15] A heat-resistant member containing the cured article according to item [13] above.

### ADVANTAGEOUS EFFECTS OF INVENTION

By the invention, healing ability and remoldability as well as dismantlability can be imparted to a cured article formed from the curable resin composition, and further such a cured article suffers no weight reduction when remolded and can contribute to an increase of the life of the cured article and a reduction of waste.

### DESCRIPTION OF EMBODIMENTS

Hereinbelow, aspects for carrying out the invention will be described in detail. The invention is not limited to the embodiment described below, and it should be understood that the design can be arbitrarily modified and implemented based on the general knowledge of those skilled in the art within a range not departing from the gist of the invention.

The glycidyl group-containing compound as one aspect of the invention includes a conjugated diene structure (A) having at least one glycidyl group and a dienophile structure (B) having at least one glycidyl group, in which the conjugated diene structure (A) and the dienophile structure (B) are linked together by the Diels-Alder reaction. Note that, in the invention, the glycidyl group does not indicate only a glycidyl group having no substituent, but includes a glycidyl group having a substituent on the carbon atom. Further, the Diels-Alder reaction includes not only the Diels-Alder reaction in a narrow sense, which is a reaction of a conjugated diene structure or dienophile structure which does not contain a heteroatom, such as an oxygen atom, a nitrogen atom, or a sulfur atom, but also the Hetero-Diels-Alder reaction which is a reaction of a conjugated diene structure or dienophile structure containing a heteroatom, such as an oxygen atom, a nitrogen atom, or a sulfur atom.

The reversible bonding by the Diels-Alder reaction is a reaction in which a conjugated diene and a dienophile undergo an addition reaction to form a six-membered ring. The Diels-Alder reaction is an equilibrium reaction, and therefore the Retro-Diels-Alder reaction occurs at a predetermined temperature to cause dissociation (decrosslinking). Since the reversibility is maintained after the cured article (three-dimensional crosslinked material) is obtained, when mechanical energy, such as a flaw or external force, is applied to the cured article, the C-C bond of the Diels-Alder reaction unit is preferentially broken because the C-C bond of the Diels-Alder reaction unit has low bond energy, as compared to a general covalent bond. However, it is considered that, in the region of temperatures lower than the dissociation temperature, the equilibrium shifts in the direction of bonding, and thus the C-C bond of the Diels-Alder reaction unit forms an adduct (Diels-Alder reaction unit) again, enabling healing of the flaw or remolding.

Accordingly, the glycidyl group-containing compound as one aspect of the invention is incorporated into the crosslinked structure due to a curing reaction based on the glycidyl group of the compound, and, when an impact is applied to the resultant cured article to cause formation of a crack or pulverization, the cured article exhibits dismantlability by breakage at the reversible bonding site. However, as mentioned above, the reversible bonding reversibly reforms a bond even in the low-temperature region including room temperature, so that the cured article can exhibit the function of healing ability and remoldability. For example, even when the cured article using the glycidyl group-containing compound of the invention is pulverized, the pulverized cured article is placed in the low-temperature state including room temperature or in the warmed or heated state to cause the reversible bonding to easily heal the cured article, and further, after the cured article is pulverized, it is possible to remold the pulverized cured article. In this instance, the end on the other side of the broken site is linked to the crosslinked structure of the cured article by chemical bond, and therefore no low-molecular compound is generated. Thus, warming or heating the cured article during the remolding can suppress generation of a volatile component, making it possible to prevent a weight reduction and effectively prevent deposition of a contaminant on the mold and the like used for remolding.

The glycidyl group-containing compound as one aspect of the invention includes a conjugated diene structure (A) having at least one glycidyl group and a dienophile structure (B) having at least one glycidyl group, in which the conjugated diene structure (A) and the dienophile structure (B) are linked together. It is preferred that the glycidyl group, for example, as a glycidyl ether group or a diglycidylamino group is linked directly or through another group, such as an aromatic ring, to a compound having the below-mentioned conjugated diene structure or dienophile structure from the viewpoint of the easy availability of the raw materials. Further, the glycidyl group-containing compound may have per molecule two or more glycidyl groups having different forms, for example, a glycidyl ether group and a diglycidylamino group.

With respect to the conjugated diene structure (A), there is no particular limitation, and examples of the structures include aliphatic hydrocarbon skeletons, such as a 1,3-butadiene skeleton; aromatic hydrocarbon skeletons, such as anthracene; cyclic dienes, such as cyclopentadiene and dicyclopentadiene; and heterocyclic skeletons, such as an imidazole ring, a furan ring, a triazine ring, a thiophene ring, and a pyrrole ring. In the invention, it is essential that the compound having the above skeleton or ring have therein a glycidyl group, but the compound may have other various substituents as long as the Diels-Alder reaction is not inhibited. The number of glycidyl group per molecule is not limited to one, but may be two or more, and, from the viewpoint of the availability and easy synthesis of the compound and the balance between reactivity of the compound used in the curable resin composition and dismantlability and remoldability of the cured article, the number of glycidyl group(s) per molecule is preferably 1 to 3, more preferably 1 to 2.

Further, with respect to the dienophile structure (B), there is no particular limitation, and examples thereof include compounds having a maleimide group, an acryloyl group, a vinyl ketone group, an acetylene group, an allyl group, a diazo group, a nitro group, a benzoquinone skeleton, or the like. In the invention, it is essential that the compound having the above functional group have therein a glycidyl group, but the compound may have other various substituents as long as the Diels-Alder reaction is not inhibited. Note that the number of glycidyl group per molecule is not limited to one, but may be two or more, and, from the viewpoint of the availability and easy synthesis of the compound and the balance between reactivity of the compound used in the curable resin composition and dismantlability and remoldability of the cured article, the number of glycidyl group(s) per molecule is preferably 1 to 3, more preferably 1 to 2. The compound having the functional group may contain various substituents or structures as long as the Diels-Alder reaction is not inhibited.

With respect to the glycidyl group-containing compound as one aspect of the invention, there is no particular limitation as long as the compound includes a combination of the above-mentioned conjugated diene structure (A) and dienophile structure (B), but, from the viewpoint of the reactivity of the compound in the form of a curable resin composition, the mechanical strength of the cured article obtained from the composition, and the like, the compound preferably has an epoxy equivalent in the range of 100 to 500 g/eq.

Further, from the viewpoint of the availability of the raw materials and easy synthesis of the compound, the viewpoint of the mechanical strength of the cured article, and the like, the conjugated diene structure (A) is preferably a furan-derived structure or an anthracene-derived structure, and the dienophile structure (B) is preferably a maleimide-derived structure.

Further, especially when the cured article is used in the heat resistant application, a combination of the conjugated diene structure (A) and the dienophile structure (B) is preferably such that the dissociation temperature is 100°C or higher, particularly 120°C or higher. When the cured article is used at higher temperatures, a combination of the conjugated diene structure (A) and the dienophile structure (B) is preferably such that the dissociation temperature can be 130°C or higher, that is, it is preferred that the conjugated diene structure (A) is an anthracene-derived structure and the dienophile structure (B) is a maleimide-derived structure.

For obtaining the compound in which the conjugated diene structure (A) is a furan-derived structure or an anthracene-derived structure and the dienophile structure (B) is a maleimide-derived structure, in view of the simple and easy method, preferred is a method using a furan compound or anthracene compound having on the ring a substituent having a glycidyl group and a maleimide compound having a substituent having a glycidyl group.

The glycidyl group-containing compound as one aspect of the invention can be represented by the following structural formula.

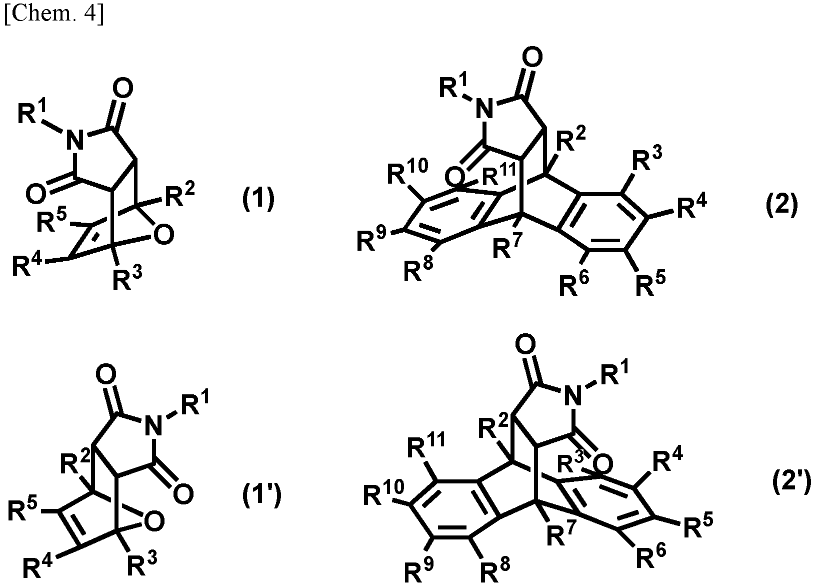

In the above formulae, R¹ is a glycidyl group or a substituent having at least one glycidyl group, and each of R² to R¹¹ is independently a hydrogen atom, a halogen atom, a hydroxyl group, a nitro group, a carboxyl group, a cyano group, an alkoxy group, an aralkyloxy group, an aryloxy group, an amino group, an amide group, an alkyloxycarbonyl group, an aryloxycarbonyl group, an alkyl group, a cycloalkyl group, an aralkyl group, an aryl group, or a glycidyl group,
and may have a substituent on the carbon atom or nitrogen atom of the alkoxy group, aralkyloxy group, aryloxy group, amino group, amide group, alkyloxycarbonyl group, aryloxycarbonyl group, alkyl group, cycloalkyl group, aralkyl group, aryl group, or glycidyl group,
with the proviso that at least one of R² to R⁵ in the formula (1) or (1') or at least one of R² to R¹¹ in the formula (2) or (2') is a glycidyl group or a group having at least one glycidyl group.

With respect to the method for producing the glycidyl group-containing compound which is an embodiment of the invention, there is no particular limitation. The glycidyl group-containing compound may be produced stepwise using a known reaction according to the intended structure, and can be obtained appropriately using commercially available raw materials in combination. A representative synthesis method is described below.

The Diels-Alder reaction, in which a conjugated diene and a dienophile undergo an addition reaction to form a six-membered ring, is an equilibrium reaction, and, with respect to the Diels-Alder reaction, it has been widely known that, at temperatures higher than the temperature at which the addition reaction proceeds, the retro-Diels-Alder reaction proceeds as a reverse reaction in which the addition reaction side suffers dissociation to be returned to the original conjugated diene and dienophile. The Diels-Alder reaction can be conducted using a known method. For example, the glycidyl group-containing compound can be obtained by mixing a conjugated diene compound and a dienophile compound in an equimolar amount or in such amounts that one component is in an excess amount, and heatmelting the resultant mixture or dissolving the mixture in a solvent, and stirring the mixture at room temperature to a temperature of 110°C for 1 to 24 hours, and subjecting the resultant reaction mixture as such to filtration or evaporation of the solvent without purification, or subjecting the reaction mixture to isolation and purification method generally used, such as recrystallization, reprecipitation, or chromatography.

For obtaining the glycidyl group-containing compound in which the conjugated diene structure (A) is a furan-derived structure, a furan compound having a hydroxyl group or an amino group which is a precursor of the glycidyl group is preferably used, and specifically, any of the compounds of the formulae shown below can be used. The hydroxyl group in the compound can be changed to a glycidyl ether group by, for example, the known method described in the Examples below, and the amino group can be similarly changed to a diglycidylamino group. Note that in the compound having a benzene ring or a naphthalene ring, the hydroxyl group or amino group may be bonded to the ring at any position.

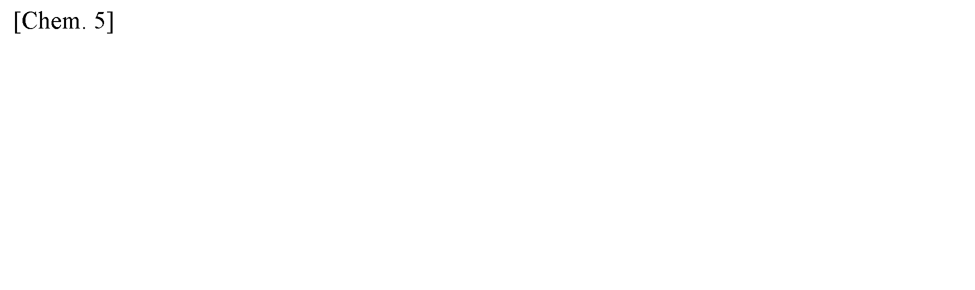

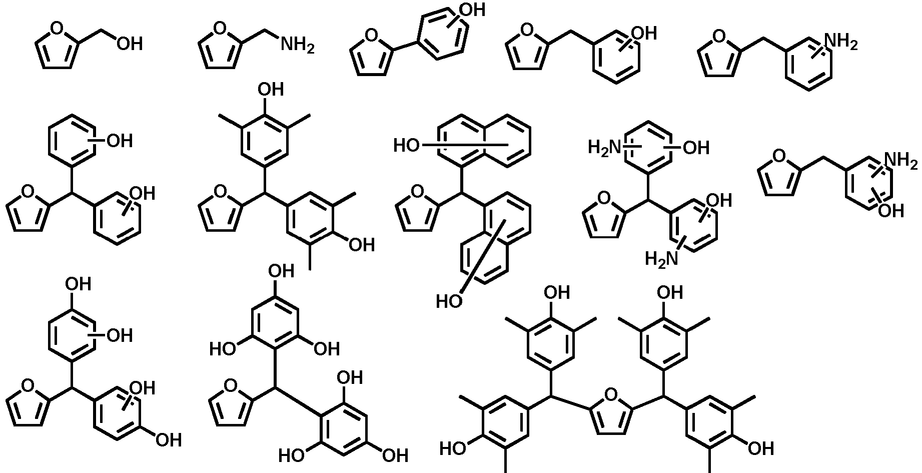

Among the compounds of the formulae shown above, the below-shown compounds are especially preferred from the viewpoint of the reactivity, physical properties of the cured article and the balance between the dismantlability, healing ability, and remoldability.

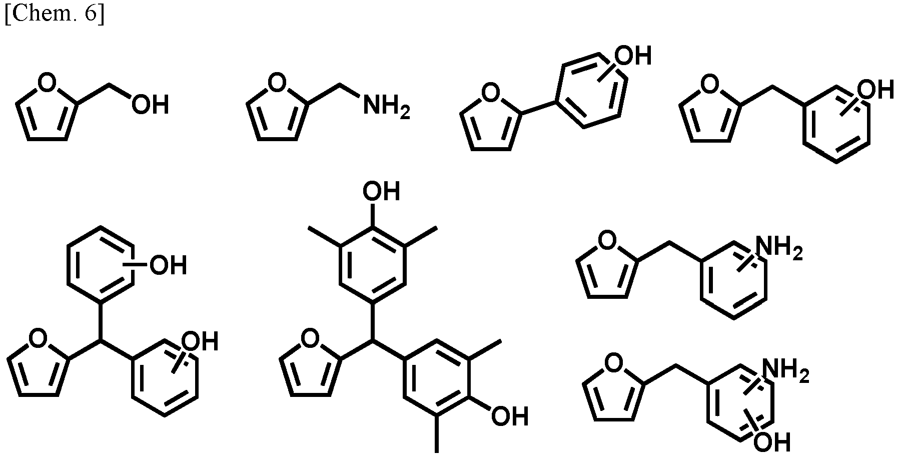

For obtaining the glycidyl group-containing compound in which the conjugated diene structure (A) is an anthracene-derived structure, an anthracene compound having a hydroxyl group or an amino group which is a precursor of the glycidyl group is preferably used, and specifically, any of the compounds of the formulae shown below can be used. Of these, 9-(4-hydroxybenzyl)-10-(4-hydroxyphenyl)anthracene and hydroxyanthracene are preferred in view of excellent curing properties, and 9-(4-hydroxybenzyl)-10-(4-hydroxyphenyl)anthracene and monohydroxyanthracene are especially preferred in view of the reactivity, physical properties of the cured article, and balance between the healing ability and remoldability. The hydroxyl group in the compound can be changed to a glycidyl ether group by, for example, the known method described in the Examples below, and the amino group can be similarly changed to a diglycidylamino group.

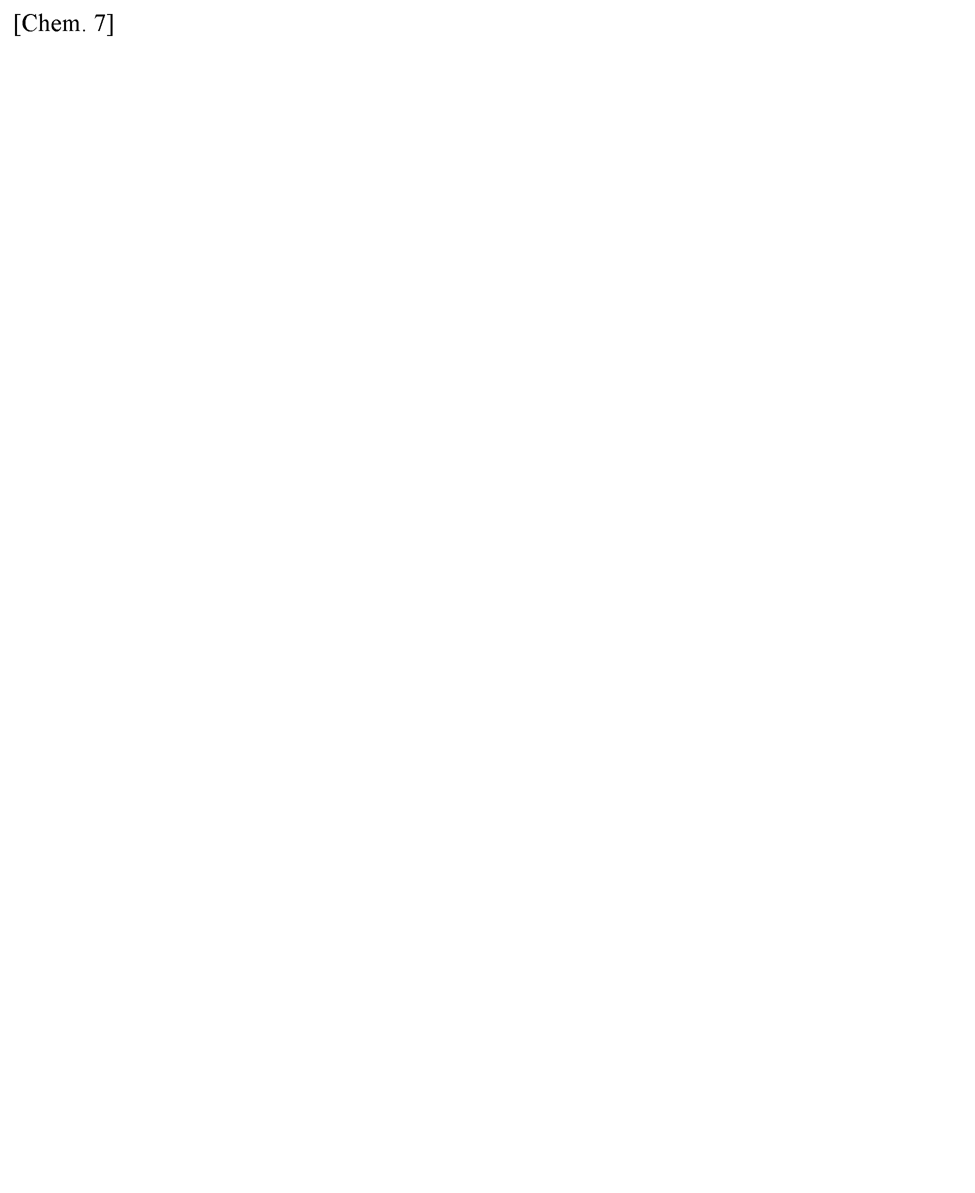

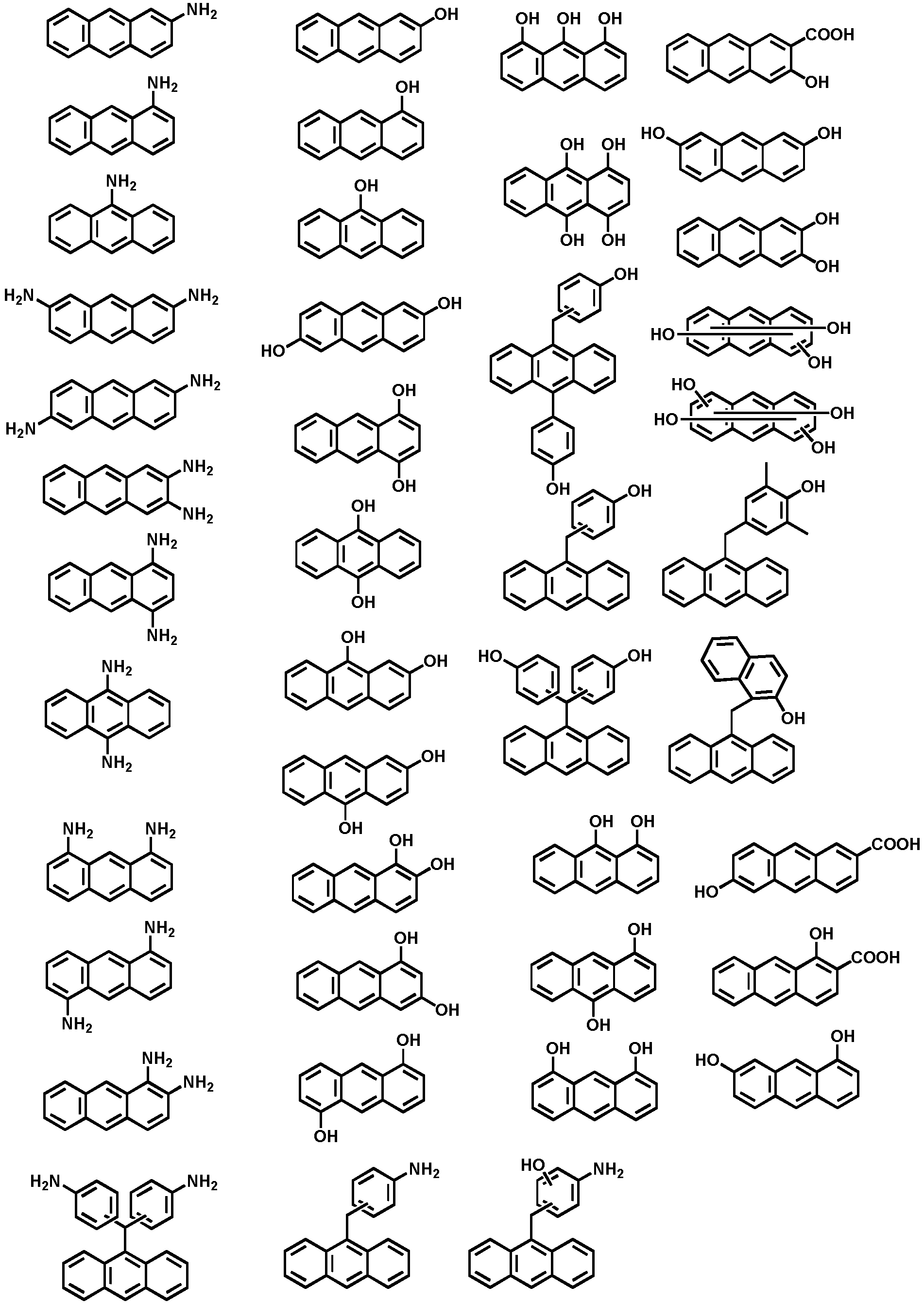

For obtaining the glycidyl group-containing compound in which the dienophile structure (B) is a maleimide-derived structure, a maleimide compound having a hydroxyl group or an amino group which is a precursor of the glycidyl group is preferably used as a raw material. As examples of the maleimide compounds having a hydroxyl group or an amino group, there can be mentioned the compounds of the formulae shown below. Of these, hydroxyphenylmaleimide is preferred in view of the curing properties when subjected to glycidyl-etherification, and monohydroxyphenylmaleimide is an especially preferred raw material in view of the reactivity, physical properties of the cured article, and balance between the healing ability and remoldability. Among the monohydroxyphenylmaleimide, from the viewpoint of the heat resistance of the cured article, parahydroxyphenylmaleimide is especially preferred. The hydroxyl group in the compound can be changed to a glycidyl ether group by, for example, the known method described in the Examples below, and the amino group can be similarly changed to a diglycidylamino group.

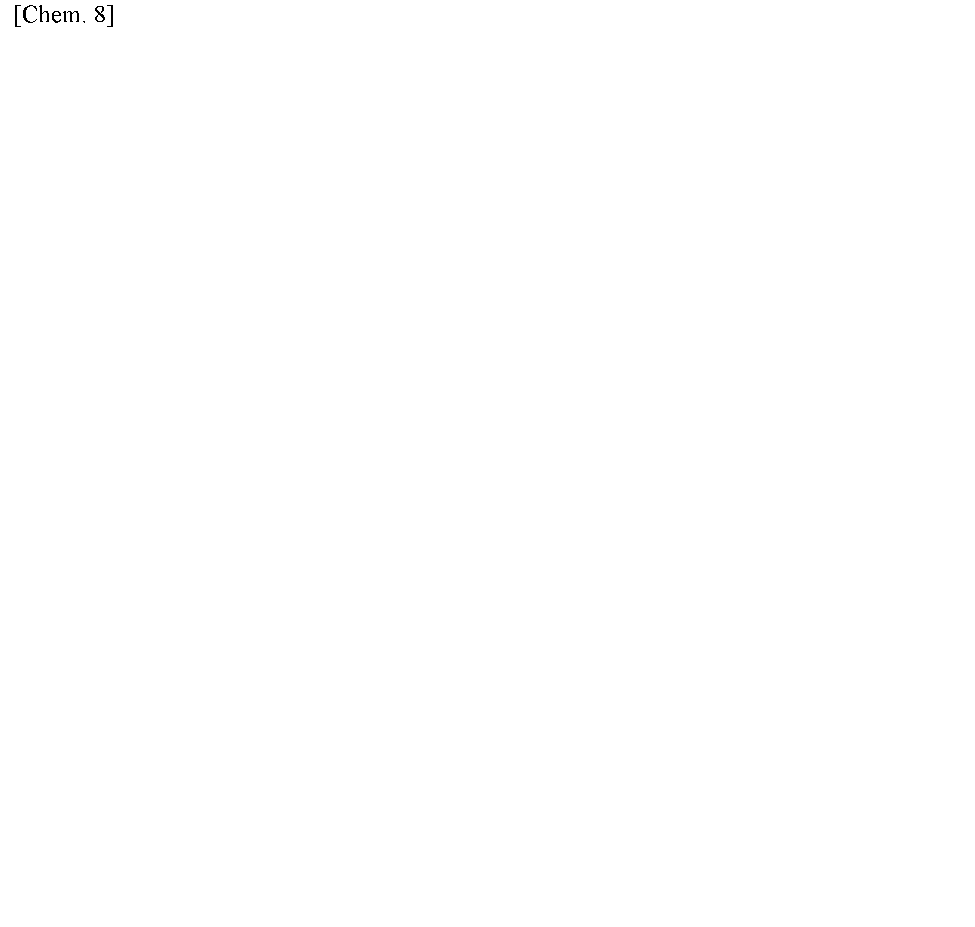

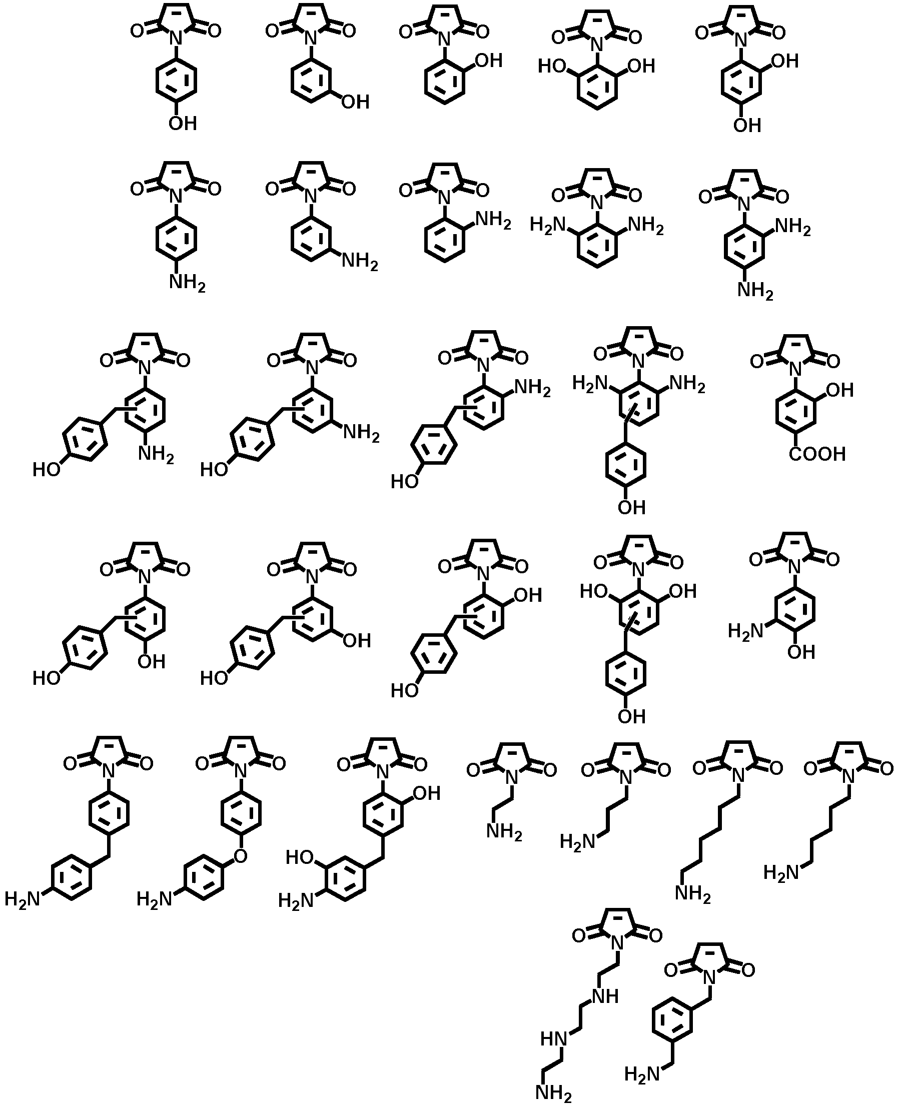

Note that the structures of the furan compound, anthracene compound, and maleimide compound include those having, as a substituent, independently a hydrogen atom, a halogen atom, an alkoxy group, an aralkyloxy group, an aryloxy group, a nitro group, an amide group, an alkyloxycarbonyl group, an aryloxycarbonyl group, a cyano group, an alkyl group, a cycloalkyl group, an aralkyl group, or an aryl group. Further, in the structure of the compounds of the formulae shown above, the alkoxy group, aralkyloxy group, aryloxy group, carboxyl group, alkyloxycarbonyl group, aryloxycarbonyl group, alkyl group, cycloalkyl group, aralkyl group, and aryl group include those further having various substituents bonded to the carbon atom or nitrogen atom thereof.

The glycidyl group-containing compound of the invention is used in combination with a compound (I) having reactivity with the glycidyl group-containing compound, so that a curable resin composition can be obtained. The curable resin composition can be advantageously used in various electric or electronic member applications, such as an adhesive, a coating composition, a photoresist, a printed wiring board, and a semiconductor encapsulation material.

Examples of the compound (I) having reactivity with the glycidyl group-containing compound include various known curing agents for epoxy resin, such as an amine compound, an acid anhydride, an amide compound, a phenolic hydroxyl group-containing compound, a carboxylic acid compound, and a thiol compound. The curing agent can be appropriately selected according to the intended physical properties of the cured article, and, particularly from the viewpoint of the mechanical strength, adhesion to a substrate, and the like, a hydroxyl group-containing compound is preferably used.

Examples of the amine compounds include aliphatic amine compounds, such as trimethylenediamine, ethylenediamine, N,N,N',N'-tetramethylethylenediamine, pentamethyldiethylenetriamine, triethylenediamine, dipropylenediamine, N,N,N',N'-tetramethylpropylenediamine, tetramethylenediamine, pentanediamine, hexamethylenediamine, trimethylhexamethylenediamine, N,N,N',N'-tetramethylhexamethylenediamine, N,N-dimethylcyclohexylamine, diethylenetriamine, triethylenetetramine, tetraethylenepentamine, dimethylaminopropylamine, diethylaminopropylamine, dibutylaminopropylamine, 1,4-diazabicyclo(2,2,2)octane(triethylenediamine), polyoxyethylenediamine, polyoxypropylenediamine, bis(2-dimethylaminoethyl) ether, dimethylaminoethoxyethoxyethanol, triethanolamine, dimethylaminohexanol, benzylmethylamine, dimethylbenzylamine, m-xylenediamine, and α-methylbenzylmethylamine;

alicyclic or heterocyclic amine compounds, such as piperidine, piperazine, menthanediamine, isophoronediamine, methylmorpholine, ethylmorpholine, N,N',N"-tris(dimethylaminopropyl)hexahydro-s-triazine, 3,9-bis(3-aminopropyl)-2,4,8,10-tetraoxyspiro(5,5)undecane adduct, N-aminoethylpiperazine, trimethylaminoethylpiperazine, bis(4-aminocyclohexyl)methane, N,N'-dimethylpiperazine, and 1,8-diazabicyclo-[5.4.0]-undecene (DBU);
aromatic amine compounds, such as o-phenylenediamine, m-phenylenediamine, p-phenylenediamine, diaminodiphenylmethane, diaminodiphenyl sulfone, pyridine, and picoline; and
modified amine compounds, such as epoxy compound addition polyamine, Michael addition polyamine, Mannich addition polyamine, thiourea addition polyamine, ketone hindered polyamine, dicyandiamide, guanidine, an organic acid hydrazide, diaminomaleonitrile, amine imide, a boron trifluoride-piperidine complex, and a boron trifluoride-monoethylamine complex.

Examples of the acid anhydrides include phthalic anhydride, trimellitic anhydride, pyromellitic anhydride, maleic anhydride, maleic anhydride polypropylene glycol, tetrahydrophthalic anhydride, methyltetrahydrophthalic anhydride, methylnadic anhydride, hexahydrophthalic anhydride, and methylhexahydrophthalic anhydride.

Examples of the phenolic hydroxyl group-containing compounds include bisphenols, such as bis(4-hydroxyphenyl)methane, 2,2-bis(4-hydroxyphenyl)propane, 2,2-bis(3-methyl-4-hydroxyphenyl)propane, 1,1-bis(4-hydroxyphenyl)cyclohexane, 1,1-bis(4-hydroxyphenyl)-1-phenylethane, and bis(4-hydroxyphenyl) sulfone; and polyhydric phenolic compounds, such as a phenolic novolak resin, a cresol novolak resin, an aromatic hydrocarbon formaldehyde resin-modified phenolic resin, a dicyclopentadiene phenol addition type resin, a phenolaralkyl resin (Zylock resin), a naphtholaralkyl resin, a trimethylolmethane resin, a tetraphenylolethane resin, a naphthol novolak resin, a naphthol-phenol copolycondensation novolak resin, a naphthol-cresol copolycondensation novolak resin, a biphenyl-modified phenolic resin (polyhydric phenolic compound having phenol nuclei linked through a bismethylene group), a biphenyl-modified naphthol resin (polyhydric naphthol compound having phenol nuclei linked through a bismethylene group), an aminotriazine-modified phenolic resin (polyhydric phenolic compound having phenol nuclei linked through melamine, benzoguanamine, or the like), and an alkoxy group-containing aromatic ring-modified novolak resin (polyhydric phenolic compound having phenol nuclei and an alkoxy group-containing aromatic ring linked through formaldehyde).

Examples of the amide compounds include dicyandiamide and polyamide-amine. Examples of the polyamide-amines include those obtained by, for example, reacting an aliphatic dicarboxylic acid, such as succinic acid, glutaric acid, adipic acid, pimelic acid, suberic acid, or azelaic acid, or a carboxylic acid compound, such as a fatty acid or dimer acid, and an aliphatic polyamine or a polyamine having a polyoxyalkylene chain, or the like.

Examples of the carboxylic acid compounds include carboxylic acid polymers, such as carboxylic acid-terminal polyester, polyacrylic acid, and maleic acid-modified polypropylene glycol.

The thiol compound is preferably a compound having two or more thiol groups per two molecules. Examples of such thiol compounds include 3,3'-dithiodipropionic acid, trimethylolpropane tris(thioglycolate), pentaerythritol tetrakis(thioglycolate), ethylene glycol dithioglycolate, 1,4-bis(3-mercaptobutyryloxy)butane, tris[(3-mercaptopropionyloxy)-ethyl]-isocyanurate, trimethylolpropane tris(3-mercaptopropionate), pentaerythritol tetrakis(3-mercaptopropionate), pentaerythritol tetrakis(3-mercaptobutyrate), dipentaerythritol hexakis(3-mercaptopropionate), 1,3,4,6-tetrakis(2-mercaptoethyl) glycoluril, 4-butanedithiol, 1,6-hexanedithiol, and 1,10-decanedithiol.

When using these curing agents, one kind of curing agent may be used alone or two or more kinds thereof may be mixed. In the application of underfill material and the like or general coating composition application, the amine compound, carboxylic acid compound, and/or acid anhydride compound is preferably used. Further, in the adhesive or flexible wiring board application, the amine compound, particularly, dicyandiamide is preferred in view of the operation properties, curing properties, and long-term stability. Further, in the semiconductor encapsulation material application, in view of the heat resistance of the cured article, the phenolic compound of a solid type is preferred. Further, in the battery application, the aliphatic amine or thiol compound is preferred in view of low-temperature curing.

Further, in the curable resin composition of the invention, an additional epoxy resin other than the glycidyl group-containing compound of the invention can be used in combination in such an amount that the effects of the invention are not sacrificed.

Examples of the additional epoxy resins include liquid epoxy resins, such as a bisphenol A epoxy resin, a bisphenol F epoxy resin, a bisphenol S epoxy resin, a bisphenol AD epoxy resin, a polyhydroxybenzene epoxy resin, a polyhydroxynaphthalene epoxy resin, a biphenyl epoxy resin, and a tetramethylbiphenyl epoxy resin; brominated epoxy resins, such as a brominated phenolic novolak epoxy resin; and a solid bisphenol A epoxy resin, a phenolic novolak epoxy resin, a cresol novolak epoxy resin, a triphenylmethane epoxy resin, a tetraphenylethane epoxy resin, a dicyclopentadiene-phenol addition reaction type epoxy resin, a phenolaralkyl epoxy resin, a phenylene ether epoxy resin, a naphthylene ether epoxy resin, a naphthol novolak epoxy resin, a naphtholaralkyl epoxy resin, a naphthol-phenol copolycondensation novolak epoxy resin, a naphthol-cresol copolycondensation novolak epoxy resin, an aromatic hydrocarbon formaldehyde resin-modified phenolic resin type epoxy resin, and a biphenyl-modified novolak epoxy resin, and one kind of these epoxy resins may be used alone or two or more kinds thereof may be used in combination, and are preferably appropriately selected according to the intended use, physical properties of the cured article, and the like.

Of these, an epoxy resin having an epoxy equivalent of 100 to 10,000 g/eq is preferably used from the viewpoint of achieving excellent balance between the curing properties and crosslinking density of the obtained cured article, and particularly, an epoxy resin which is represented by the following formula (3), and which has an epoxy equivalent of 500 to 10,000 g/eq is preferably used: 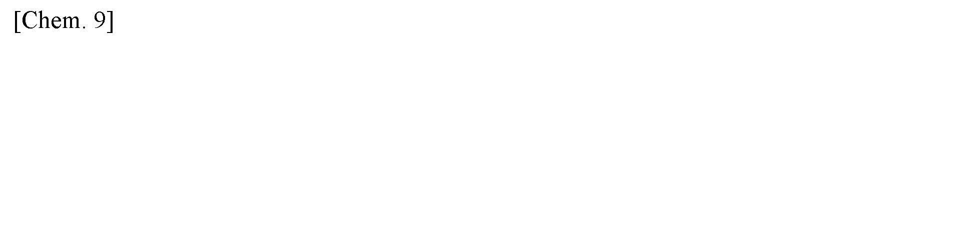 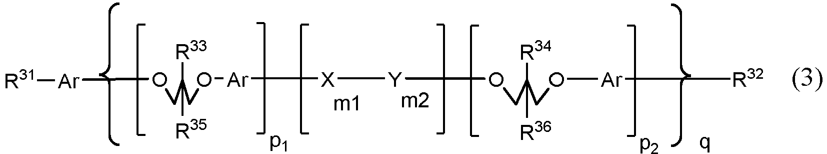 in the formula (3), each Ar is independently a structure having an aromatic ring which is unsubstituted or which has a substituent,
X is a structural unit represented by the following formula (3-1), Y is a structural unit represented by the following formula (3-2): 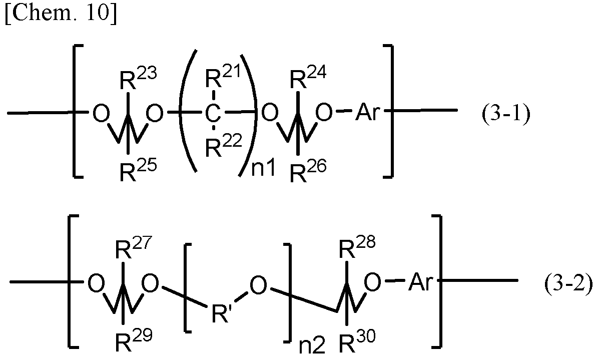 in the formulae (3-1) and (3-2), Ar is as defined above,
   each of R²¹ and R²² is independently a hydrogen atom, a methyl group, or an ethyl group, R' is a divalent hydrocarbon group having 2 to 12 carbon atoms,
   each of R²³, R²⁴, R²⁷, and R²⁸ is independently a hydroxyl group, a glycidyl ether group, or a 2-methylglycidyl ether group,
   each of R²⁵, R²⁶, R²⁹, and R³⁰ is independently a hydrogen atom or a methyl group,
   n₁ is an integer of 4 to 16, and
   n₂ represents an average of the numbers of the repeating units and is 2 to 30,
each of R³¹ and R³² is independently a glycidyl ether group or a 2-methylglycidyl ether group,
each of R³³ and R³⁴ is independently a hydroxyl group, a glycidyl ether group, or a 2-methylglycidyl ether group,
R³⁵ and R³⁶ are a hydrogen atom or a methyl group, and
m1, m2, p1, p2, and q are an average of the numbers of repetition,
in which each of m1 and m2 is independently 0 to 25, and m1 and m2 satisfy the relationship: m1 + m2 ≥ 1,
each of p1 and p2 is independently 0 to 5, and
q is 0.5 to 5,
in which the bonding of the structural unit X represented by the formula (3-1) above and the structural unit Y represented by the formula (3-2) above is in a random or block form, and the total number of structural units X and the total number of structural units Y present per molecule are m1 and m2, respectively.

With respect to the epoxy resin, for example, an epoxy resin represented by the formula below can be used. By using such an epoxy resin, the epoxy resin cured article is improved in the effect for healing ability and remoldability, achieving excellent balance between the flexibility and toughness. 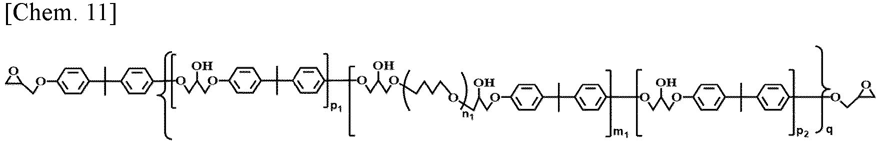 In the formula (5), each of p₁, p₂, q, m₁, and n₁ is independently an average of the numbers of repetition, in which p₁ is 0 to 5, p₂ is 0 to 5, q is 0.5 to 5, m₁ is 0 to 25, and n₁ is 2 to 30.

The epoxy resin represented by the general formula (3) above can be solely used to form the curable resin composition, but, from the viewpoint of imparting further excellent flexibility to cause the cured article to easily exhibit dismantlability, it is preferred that an epoxy resin having an epoxy equivalent of 100 to 300 g/eq is further used in combination with the epoxy resin of the formula (3).

With respect to the structure of the epoxy resin which can be used in combination with the epoxy resin of the formula (3), there is no particular limitation as long as the epoxy resin has an epoxy equivalent in the range of from 100 to 300 g/eq. Examples of such epoxy resins include liquid epoxy resins, such as a bisphenol A epoxy resin, a bisphenol F epoxy resin, a bisphenol S epoxy resin, a bisphenol AD epoxy resin, a polyhydroxybenzene epoxy resin, a polyhydroxynaphthalene epoxy resin, a biphenyl epoxy resin, and a tetramethylbiphenyl epoxy resin; brominated epoxy resins, such as a brominated phenolic novolak epoxy resin; and a solid bisphenol A epoxy resin, a phenolic novolak epoxy resin, a cresol novolak epoxy resin, a triphenylmethane epoxy resin, a tetraphenylethane epoxy resin, a dicyclopentadiene-phenol addition reaction type epoxy resin, a phenolaralkyl epoxy resin, a phenylene ether epoxy resin, a naphthylene ether epoxy resin, a naphthol novolak epoxy resin, a naphtholaralkyl epoxy resin, a naphthol-phenol copolycondensation novolak epoxy resin, a naphthol-cresol copolycondensation novolak epoxy resin, an aromatic hydrocarbon formaldehyde resin-modified phenolic resin type epoxy resin, and a biphenyl-modified novolak epoxy resin, and one kind of these epoxy resins may be used alone or two or more kinds thereof may be used in combination, and are preferably appropriately selected according to the intended use, physical properties of the cured article, and the like.

Of these, among the liquid epoxy resins, such as a bisphenol A epoxy resin, a bisphenol F epoxy resin, a bisphenol S epoxy resin, a bisphenol AD epoxy resin, a polyhydroxybenzene epoxy resin, a polyhydroxynaphthalene epoxy resin, a biphenyl epoxy resin, and a tetramethylbiphenyl epoxy resin, an epoxy resin having an epoxy equivalent of 100 to 300 g/eq is preferably used, and, among the bisphenol A epoxy resin, bisphenol F epoxy resin, bisphenol S epoxy resin, and bisphenol AD epoxy resin, an epoxy resin having an epoxy equivalent of 100 to 300 g/eq is especially preferably used.

With respect to the ratio of the epoxy resin represented by the general formula (3) above and the epoxy resin having an epoxy equivalent of 100 to 300 g/eq, there is no particular limitation, but, from the viewpoint of facilitating phase separation in the cured article, the mass ratio of the former and the latter is 97:3 to 3:97, preferably 10:90 to 90:10, especially preferably 80:20 to 20:80. Phase separation in the cured article causes an "islandin-sea" structure, and the cured article can achieve both the adhesion properties and stress relaxation ability, and exhibits high adhesive force particularly in a wide region of temperatures, and further has an effect on reducing the molding shrinkage rate before and after heat-curing the resin composition.

In the curable resin composition of the invention, the concentration of the reversible bonding is preferably 0.10 mmol/g or more, based on the total mass of the curable components of the curable resin composition. By virtue of such a construction, the cured article obtained from the curable resin composition has both further improved healing ability and remoldability. The concentration of the reversible bonding is more preferably 0.10 to 3.00 mmol/g, further more preferably 0.15 to 2.00 mmol/g. Note that the concentration of the reversible bonding can be appropriately selected according to the glass transition temperature of the intended cured article, which is defined by a tanδ peak top measured by means of a dynamic mechanical analysis (DMA) measurement apparatus, or the like. For example, when using the glass transition temperature as a yardstick, the cured article having a glass transition temperature of about room temperature likely exhibits satisfactory healing ability and remoldability function even on the lower concentration side of the preferred range. Meanwhile, the intended cured article having a glass transition temperature of higher than 100°C as a yardstick likely exhibits the function on the higher concentration side of the preferred range. However, in the region of temperatures higher than the glass transition temperature measured by DMA, generally, molecular motion is high, and, even when the concentration of the glycidyl group-containing compound is low, it is likely that satisfactory healing ability and remoldability function is exhibited and hence, for example, by appropriately adjusting the aging temperature for healing or the heating temperature for remolding, it is possible to control the effect of exhibiting healing ability and remoldability function. Thus, the relationship between the glass transition temperature of the cured article and the concentration of the reversible bonding is not limited to those mentioned above.

With respect to the ratio of the total of the glycidyl groups and the total of the active groups capable of reacting with the glycidyl group in the curable resin composition of the invention, there is no particular limitation, but, in view of excellent mechanical properties of the obtained cured article and the like, it is preferred that the amount of the active group capable of reacting with the glycidyl group is 0.4 to 1.5 equivalents, relative to one equivalent of the total of the glycidyl groups in the resin composition.

Further, the curable resin composition may contain a curing accelerator. With respect to the curing accelerator, various types of curing accelerators can be used, and examples thereof include an urea compound, a phosphorus compound, a tertiary amine, an imidazole, an imidazoline, an organic acid metal salt, a Lewis acid, and an amine complex salt. When used in the adhesive application, in view of excellent workability and low-temperature curing properties, an urea compound, especially 3-(3,4-dichlorophenyl)-1,1-dimethylurea (DCMU) is preferred. When used in the semiconductor encapsulation material application, in view of excellent curing properties, heat resistance, electric properties, reliability resistance to moisture, and the like, triphenylphosphine is preferred as a phosphorus compound, and 1,8-diazabicyclo-[5.4.0]-undecene is preferred as a tertiary amine.

Examples of the phosphorus compounds include alkylphosphines, such as ethylphosphine and butylphosphine; primary phosphines, such as phenylphosphine; dialkylphosphines, such as dimethylphosphine and dipropylphosphine; secondary phosphines, such as diphenylphosphine and methylethylphosphine; and tertiary phosphines, such as trimethylphosphine, triethylphosphine, and triphenylphosphine.

Examples of the imidazoles include imidazole, 1-methylimidazole, 2-methylimidazole, 3-methylimidazole, 4-methylimidazole, 5-methylimidazole, 1-ethylimidazole, 2-ethylimidazole, 3-ethylimidazole, 4-ethylimidazole, 5-ethylimidazole, 1-n-propylimidazole, 2-n-propylimidazole, 1-isopropylimidazole, 2-isopropylimidazole, 1-n-butylimidazole, 2-n-butylimidazole, 1-isobutylimidazole, 2-isobutylimidazole, 2-undecyl-1H-imidazole, 2-heptadecyl-1H-imidazole, 1,2-dimethylimidazole, 1,3-dimethylimidazole, 2,4-dimethylimidazole, 2-ethyl-4-methylimidazole, 1-phenylimidazole, 2-phenyl-1H-imidazole, 4-methyl-2-phenyl-1H-imidazole, 2-phenyl-4-methylimidazole, 1-benzyl-2-methylimidazole, 1-benzyl-2-phenylimidazole, 1-cyanoethyl-2-methylimidazole, 1-cyanoethyl-2-ethyl-4-methylimidazole, 1-cyanoethyl-2-undecylimidazole, 1-cyanoethyl-2-phenylimidazole, a 2-phenylimidazole isocyanuric acid addition product, a 2-methylimidazole isocyanuric acid addition product, 2-phenyl-4,5-dihydroxymethylimidazole, 2-phenyl-4-methyl-5-hydroxymethylimidazole, 1-cyanoethyl-2-phenyl-4,5-di(2-cyanoethoxy)methylimidazole, 1-dodecyl-2-methyl-3-benzylimidazolium chloride, and 1-benzyl-2-phenylimidazole hydrochloride.

Examples of the imidazoline compounds include 2-methylimidazoline and 2-phenylimidazoline.

Examples of the urea compounds include p-chlorophenyl-N,N-dimethylurea, 3-phenyl-1,1-dimethylurea, 3-(3,4-dichlorophenyl)-N,N-dimethylurea, and N-(3-chloro-4-methylphenyl)-N',N'-dimethylurea.

Further, in the curable resin composition of the invention, an additional thermosetting resin or thermoplastic resin may be used as long as the effects of the invention are not sacrificed.

Examples of additional thermosetting resins include a cyanate ester resin, a resin having a benzoxazine structure, an active ester resin, a vinylbenzyl compound, an acrylic compound, and a copolymer of styrene and maleic anhydride. When the above-mentioned additional thermosetting resin is used, there is no particular limitation with respect to the amount of the additional thermosetting resin used as long as the effects of the invention are not sacrificed, but the amount of the additional thermosetting resin used is preferably in the range of 1 to 50 parts by mass, relative to 100 parts by mass of the curable resin composition.

Examples of the cyanate ester resins include a bisphenol A cyanate ester resin, a bisphenol F cyanate ester resin, a bisphenol E cyanate ester resin, a bisphenol S cyanate ester resin, a bisphenol sulfide cyanate ester resin, a phenylene ether cyanate ester resin, a naphthylene ether cyanate ester resin, a biphenyl cyanate ester resin, a tetramethylbiphenyl cyanate ester resin, a polyhydroxynaphthalene cyanate ester resin, a phenolic novolak cyanate ester resin, a cresol novolak cyanate ester resin, a triphenylmethane cyanate ester resin, a tetraphenylethane cyanate ester resin, a dicyclopentadiene-phenol addition reaction type cyanate ester resin, a phenolaralkyl cyanate ester resin, a naphthol novolak cyanate ester resin, a naphtholaralkyl cyanate ester resin, a naphthol-phenol copolycondensation novolak cyanate ester resin, a naphthol-cresol copolycondensation novolak cyanate ester resin, an aromatic hydrocarbon formaldehyde resin-modified phenolic resin type cyanate ester resin, a biphenyl-modified novolak cyanate ester resin, and an anthracene cyanate ester resin. One kind of these resins may be used alone or two or more kinds thereof may be used in combination.

Of these cyanate ester resins, in view of obtaining a cured article having especially excellent heat resistance, a bisphenol A cyanate ester resin, a bisphenol F cyanate ester resin, a bisphenol E cyanate ester resin, a polyhydroxynaphthalene cyanate ester resin, a naphthylene ether cyanate ester resin, or a novolak cyanate ester resin is preferably used, and, in view of obtaining a cured article having excellent dielectric properties, a dicyclopentadiene-phenol addition reaction type cyanate ester resin is preferred.

With respect to the resin having a benzoxazine structure, there is no particular limitation, but examples of such resins include a reaction product of bisphenol F, formalin, and aniline (F-a type benzoxazine resin), a reaction product of diaminodiphenylmethane, formalin, and phenol (P-d type benzoxazine resin), a reaction product of bisphenol A, formalin, and aniline, a reaction product of dihydroxydiphenyl ether, formalin, and aniline, a reaction product of diaminodiphenyl ether, formalin, and phenol, a reaction product of a dicyclopentadiene-phenol addition type resin, formalin, and aniline, a reaction product of phenolphthalein, formalin, and aniline, and a reaction product of diphenyl sulfide, formalin, and aniline. One kind of these resins may be used alone or two or more kinds thereof may be used in combination.

With respect to the active ester resin, there is no particular limitation, but generally, a compound having two or more ester groups having high reaction activity per molecule, such as a phenol ester, a thiophenol ester, an N-hydroxyamine ester, or an ester of a heterocyclic hydroxy compound, is preferably used. With respect to the active ester resin, preferred is an active ester resin which is obtained by a condensation reaction of a carboxylic acid compound and/or a thiocarboxylic acid compound and a hydroxy compound and/or a thiol compound. Particularly, from the viewpoint of the improvement of heat resistance, preferred is an active ester resin obtained from a carboxylic acid compound or a halide thereof and a hydroxy compound, and more preferred is an active ester resin obtained from a carboxylic acid compound or a halide thereof and a phenolic compound and/or a naphthol compound. Examples of carboxylic acid compounds include benzoic acid, acetic acid, succinic acid, maleic acid, itaconic acid, phthalic acid, isophthalic acid, terephthalic acid, and pyromellitic acid, and a halide thereof. Examples of phenolic compounds or naphthol compounds include hydroquinone, resorcin, bisphenol A, bisphenol F, bisphenol S, dihydroxydiphenyl ether, phenolphthalein, methylated bisphenol A, methylated bisphenol F, methylated bisphenol S, phenol, o-cresol, m-cresol, p-cresol, catechol, α-naphthol, β-naphthol, 1,5-dihydroxynaphthalene, 1,6-dihydroxynaphthalene, 2,6-dihydroxynaphthalene, dihydroxybenzophenone, trihydroxybenzophenone, tetrahydroxybenzophenone, phloroglucin, benzenetriol, and a dicyclopentadiene-phenol addition type resin.

With respect to the active ester resin, specifically, preferred are an active ester resin having a dicyclopentadiene-phenol addition structure, an active ester resin having a naphthalene structure, an active ester resin which is an acetylation product of phenolic novolak, an active ester resin which is a benzoylation product of phenolic novolak, and the like, and especially, in view of advantageously improving the peel strength, an active ester resin having a dicyclopentadiene-phenol addition structure and an active ester resin having a naphthalene structure are more preferred.

Further, various novolak resins, an addition polymerization resin of an alicyclic diene compound, such as dicyclopentadiene, and a phenolic compound, a modified novolak resin of a phenolic hydroxyl group-containing compound and an alkoxy group-containing aromatic compound, a phenolaralkyl resin (Zylock resin), a naphtholaralkyl resin, a trimethylolmethane resin, a tetraphenylolethane resin, a biphenyl-modified phenolic resin, a biphenyl-modified naphthol resin, an aminotriazine-modified phenolic resin, and various vinyl polymers can be further used.

As more specific examples of the various novolak resins, there can be mentioned a polymer obtained by subjecting a phenolic hydroxyl group-containing compound, such as phenol, phenylphenol, resorcinol, biphenyl, a bisphenol, e.g., bisphenol A or bisphenol F, naphthol, or dihydroxynaphthalene, and an aldehyde compound to reaction under acid catalyst conditions.

Examples of the various vinyl polymers include a homopolymer or a copolymer of a vinyl compound, such as polyhydroxystyrene, polystyrene, polyvinylnaphthalene, polyvinyl anthracene, polyvinylcarbazole, polyindene, polyacenaphthylene, polynorbornene, polycyclodecene, polytetracyclododecene, polynortricyclene, and poly(meth)acrylate.

The thermoplastic resin means a resin which can be melt-molded by heating. Specific examples of thermoplastic resins include a polyethylene resin, a polypropylene resin, a polystyrene resin, a rubber-modified polystyrene resin, an acrylonitrile-butadiene-styrene (ABS) resin, an acrylonitrile-styrene (AS) resin, a polymethyl methacrylate resin, an acrylic resin, a polyvinyl chloride resin, a polyvinylidene chloride resin, a polyethylene terephthalate resin, an ethylene-vinyl alcohol resin, a cellulose acetate resin, an ionomer resin, a polyacrylonitrile resin, a polyamide resin, a polyacetal resin, a polybutylene terephthalate resin, a polylactic acid resin, a polyphenylene ether resin, a modified polyphenylene ether resin, a polycarbonate resin, a polysulfone resin, a polyphenylene sulfide resin, a polyether imide resin, a polyether sulfone resin, a polyarylate resin, a thermoplastic polyimide resin, a polyamide-imide resin, a polyether ether ketone resin, a polyketone resin, a liquid crystalline polyester resin, a fluororesin, a syndiotactic polystyrene resin, and a cyclic polyolefin resin. One kind of these thermoplastic resins can be used alone or two or more kinds of thereof can be used in combination.

When the additional resin is used, the blending ratio of the glycidyl group-containing compound of the invention and the additional resin can be arbitrarily selected according to the use of the composition, and, from the viewpoint of inhibiting the healing ability and remoldability achieved by the invention, the amount of the additional resin is 0.5 to 100 parts by mass, relative to 100 parts by mass of the glycidyl group-containing compound of the invention.

Further, when used in the application that requires the curable resin composition of the invention to have high flame retardancy, a non-halogen flame retardant not substantially containing halogen atom may be blended into the composition.

Examples of the non-halogen flame retardants include a phosphorus flame retardant, a nitrogen flame retardant, a silicone flame retardant, an inorganic flame retardant, and an organometal salt flame retardant, and there is no particular limitation with respect to the use of the non-halogen flame retardant, and a non-halogen flame retardant can be solely used, or two or more flame retardants of the same type can be used, or flame retardants of different types can be used in combination.

With respect to the phosphorus flame retardant, any of an inorganic phosphorus flame retardant and an organic phosphorus flame retardant can be used. Examples of inorganic compounds include red phosphorus, ammonium phosphates, such as monoammonium phosphate, diammonium phosphate, triammonium phosphate, and ammonium polyphosphate, and inorganic nitrogen-containing phosphorus compounds, such as phosphoric acid amide.

Further, it is preferred that the red phosphorus has been subjected to surface treatment for the purpose of preventing the phosphorus from suffering hydrolysis or the like, and examples of methods for surface treatment include (i) a method in which red phosphorus is subjected to coating treatment with an inorganic compound, such as magnesium hydroxide, aluminum hydroxide, zinc hydroxide, titanium hydroxide, bismuth oxide, bismuth hydroxide, bismuth nitrate, or a mixture thereof, (ii) a method in which red phosphorus is subjected to coating treatment with a mixture of an inorganic compound, such as magnesium hydroxide, aluminum hydroxide, zinc hydroxide, or titanium hydroxide, and a thermosetting resin, such as a phenolic resin, and (iii) a method in which red phosphorus is subjected to double coating treatment with a thermosetting resin, such as a phenolic resin, on a film of an inorganic compound, such as magnesium hydroxide, aluminum hydroxide, zinc hydroxide, or titanium hydroxide.

Examples of the organophosphorus compounds include general-purpose organophosphorus compounds, such as a phosphate compound, a phosphonic acid compound, a phosphinic acid compound, a phosphine oxide compound, a phosphorane compound, and an organic nitrogen-containing phosphorus compound, and cyclic organophosphorus compounds, such as 9,10-dihydro-9-oxa-10-phosphaphenanthren-10-oxide, 10-(2,5-dihydroxyphenyl)-10H-9-oxa-10-phosphaphenanthren-10-oxide, and 10-(2,7-dihydroxynaphthyl)-10H-9-oxa-10-phosphaphenanthren-10-oxide, and a derivative obtained by reacting the cyclic organophosphorus compound with a compound, such as an epoxy resin or a phenolic resin.

The blended amount of the phosphorus flame retardant is appropriately selected according to the type of the phosphorus flame retardant, the other components of the resin composition, or the degree of the desired flame retardancy, and, for example, when red phosphorus is used as a non-halogen flame retardant, the blended amount of the phosphorus flame retardant is preferably in the range of 0.1 to 2.0 parts by mass, relative to 100 parts by mass of the resin composition having blended all the non-halogen flame retardant, additional filler, additive, and the like, and, when an organophosphorus compound is used as a non-halogen flame retardant, similarly, the blended amount of the phosphorus flame retardant is preferably in the range of 0.1 to 10.0 parts by mass, more preferably in the range of 0.5 to 6.0 parts by mass.

Further, when the phosphorus flame retardant is used, hydrotalcite, magnesium hydroxide, a boron compound, zirconium oxide, a black dye, calcium carbonate, zeolite, zinc molybdate, activated carbon, or the like may be used in combination with the phosphorus flame retardant.

Examples of the nitrogen flame retardants include a triazine compound, a cyanuric acid compound, an isocyanuric acid compound, and phenothiazine, and preferred are a triazine compound, a cyanuric acid compound, and an isocyanuric acid compound.

Examples of the triazine compounds include melamine, acetoguanamine, benzoguanamine, melone, melame, succinoguanamine, ethylenedimelamine, melamine polyphosphate, triguanamine, and the like; in addition, (1) aminotriazine sulfate compounds, such as guanylmelamine sulfate, melem sulfate, and melame sulfate; (2) a copolycondensation product of phenols, such as phenol, cresol, xylenol, butylphenol, or nonylphenol, melamines, such as melamine, benzoguanamine, acetoguanamine, or formguanamine, and formaldehyde; (3) a mixture of the copolycondensation product of the item (2) above and phenolic resins, such as a phenol formaldehyde condensation product; and (4) a compound obtained by further modifying the compound of the item (2) or (3) above with tung oil, isomerized linseed oil, or the like.

Examples of the cyanuric acid compounds include cyanuric acid and melamine cyanurate.

The blended amount of the nitrogen flame retardant is appropriately selected according to the type of the nitrogen flame retardant, the other components of the resin composition, or the degree of the desired flame retardancy, and, for example, is preferably in the range of 0.05 to 10 parts by mass, more preferably in the range of 0.1 to 5 parts by mass, relative to 100 parts by mass of the resin composition having blended all the non-halogen flame retardant, additional filler, additive, and the like.

Further, when the nitrogen flame retardant is used, a metal hydroxide, a molybdenum compound, or the like may be used in combination with the nitrogen flame retardant.

With respect to the silicone flame retardant, there is no particular limitation as long as the flame retardant is an organic compound containing a silicon atom, and any silicone flame retardant can be used, and examples of such silicone flame retardants include a silicone oil, a silicone rubber, and a silicone resin. The blended amount of the silicone flame retardant is appropriately selected according to the type of the silicone flame retardant, the other components of the resin composition, or the degree of the desired flame retardancy, and, for example, is preferably in the range of 0.05 to 20 parts by mass, relative to 100 parts by mass of the resin composition having blended all the non-halogen flame retardant, additional filler, additive, and the like. Further, when the silicone flame retardant is used, a molybdenum compound, alumina, or the like may be used in connection with the silicone flame retardant.

Examples of the inorganic flame retardants include a metal hydroxide, a metal oxide, a metal carbonate compound, a metal powder, a boron compound, and low melting-point glass.

Examples of the metal hydroxides include aluminum hydroxide, magnesium hydroxide, dolomite, hydrotalcite, calcium hydroxide, barium hydroxide, and zirconium hydroxide.

Examples of the metal oxides include zinc molybdate, molybdenum trioxide, zinc stannate, tin oxide, aluminum oxide, iron oxide, titanium oxide, manganese oxide, zirconium oxide, zinc oxide, molybdenum oxide, cobalt oxide, bismuth oxide, chromium oxide, nickel oxide, copper oxide, and tungsten oxide.

Examples of the metal carbonate compounds include zinc carbonate, magnesium carbonate, calcium carbonate, barium carbonate, basic magnesium carbonate, aluminum carbonate, iron carbonate, cobalt carbonate, and titanium carbonate.

Examples of the metal powders include aluminum, iron, titanium, manganese, zinc, molybdenum, cobalt, bismuth, chromium, nickel, copper, tungsten, and tin.

Examples of the boron compounds include zinc borate, zinc metaborate, barium metaborate, boric acid, and borax.

Examples of the low melting-point glass include Ceepree (Bokusui Brown Co., Ltd.), hydrated glass SiO₂-MgO-H₂O, and glassy compounds of PbO-B₂O₃ type, ZnO-P₂O₅-MgO type, P₂O₅-B₂O₃-PbO-MgO type, P-Sn-O-F type, PbO-V₂O₅-TeO₂ type, Al₂O₃-H₂O type, lead borosilicate glassy compounds, or the like.

The blended amount of the inorganic flame retardant is appropriately selected according to the type of the inorganic flame retardant, the other components of the resin composition, or the degree of the desired flame retardancy, but, for example, is preferably in the range of 0.05 to 20 parts by mass, more preferably in the range of 0.5 to 15 parts by mass, relative to 100 parts by mass of the resin composition having blended all the non-halogen flame retardant, additional filler, additive, and the like.

Examples of the organometal salt flame retardants include ferrocene, an acetylacetonato metal complex, an organometal carbonyl compound, an organocobalt salt compound, an organic sulfonic acid metal salt, and a compound in which a metal atom and an aromatic compound or heterocyclic compound are ionically bonded or coordinate-bonded.

The blended amount of the organometal salt flame retardant is appropriately selected according to the type of the organometal salt flame retardant, the other components of the resin composition, or the degree of the desired flame retardancy, and, for example, is preferably in the range of 0.005 to 10 parts by mass, relative to 100 parts by mass of the resin composition having blended all the non-halogen flame retardant, additional filler, additive, and the like.

The curable resin composition of the invention may contain a filler. Examples of fillers include inorganic fillers and organic fillers. Examples of inorganic fillers include inorganic fine particles.

Examples of inorganic fine particles include those having excellent heat resistance, such as alumina, magnesia, titania, zirconia, and silica (such as quartz, fumed silica, precipitated silica, silicic anhydride, fused silica, crystalline silica, and ultrafinely powdered amorphous silica); those having excellent thermal conductivity, such as boron nitride, aluminum nitride, alumina oxide, titanium oxide, magnesium oxide, zinc oxide, silicon oxide, and diamond; those having excellent electrical conductivity, such as a metallic filler and/or a metal coated filler using a metal simple substance or an alloy (e.g., iron, copper, magnesium, aluminum, gold, silver, platinum, zinc, manganese, or stainless steel); those having excellent barrier properties, such as minerals, e.g., mica, clay, kaolin, talc, zeolite, wollastonite, and smectite, potassium titanate, magnesium sulfate, sepiolite, zonolite, aluminum borate, calcium carbonate, titanium oxide, barium sulfate, zinc oxide, and magnesium hydroxide; those having high refractive index, such as barium titanate, zirconia oxide, and titanium oxide; those having photocatalytic properties, such as photocatalytic metals, e.g., titanium, cerium, zinc, copper, aluminum, tin, indium, phosphorus, carbon, sulfur, ruthenium, nickel, iron, cobalt, silver, molybdenum, strontium, chromium, barium, and lead, and composites of the above metal and oxides thereof; those having excellent wear resistance, such as metals, e.g., silica, alumina, zirconia, and magnesium oxide, and composites and oxides thereof; those having excellent electrical conductivity, such as metals, e.g., silver and copper, and tin oxide, and indium oxide; those having excellent insulating properties, such as silica; and those having excellent ultraviolet light screen, such as titanium oxide and zinc oxide. These inorganic fine particles can be appropriately selected according to the use, and those of a single type can be used, or those of two or more types can be used in combination. Further, the inorganic fine particles have various properties other than the properties mentioned above as examples, and thus may be appropriately selected according to the use.

For example, when silica is used as the inorganic fine particles, there is no particular limitation with respect to the silica, and known silica fine particles, such as silica in a powder form or colloidal silica, can be used. Examples of commercially available silica fine particles in a powder form include Aerosil 50, 200, manufactured by Nippon Aerosil Co., Ltd.; Sildex H31, H32, H51, H52, H121, H122, manufactured by AGC Inc.; E220A, E220, manufactured by Nippon Silica Industrial Co.; SYLYSIA 470, manufactured by Fuji Silysia Chemical Ltd.; and SG Flake, manufactured by Nippon Sheet Glass Co., Ltd.

Further, examples of commercially available colloidal silica include methanol silica sol, IPA-ST, MEK-ST, NBA-ST, XBA-ST, DMAC-ST, ST-UP, ST-OUP, ST-20, ST-40, ST-C, ST-N, ST-O, ST-50, ST-OL, manufactured by Nissan Chemical Corporation.

Surface-modified silica fine particles may be used, and, for example, there can be mentioned the silica fine particles which are surface-treated with a reactive silane coupling agent having a hydrophobic group, and the silica fine particles which are modified with a compound having (a)an (meth)acryloyl group. As examples of commercially available powder-form silica modified with a compound having (a)an (meth)acryloyl group, there can be mentioned Aerosil RM50, R711, manufactured by Nippon Aerosil Co., Ltd., and, as examples of commercially available colloidal silica modified with a compound having (a)an (meth)acryloyl group, there can be mentioned MIBK-SD, manufactured by Nissan Chemical Corporation.

With respect to the form of the silica fine particles, there is no particular limitation, and those in a spherical form, a hollow form, a porous form, a rod form, a plate form, a fiber form, or an amorphous form can be used. Further, the primary particle diameter of the silica fine particles is preferably in the range of 5 to 200 nm.

As the titanium oxide fine particles, not only a loading pigment but also an ultraviolet light-responsive photocatalyst can be used, and, for example, anatase titanium oxide, rutile titanium oxide, or brookite titanium oxide can be used. Further, there can be used titanium oxide particles which have a crystal structure of titanium oxide doped with a hetero-element and which are designed so as to respond to a visible light. As an element for doping titanium oxide, an anionic element, such as nitrogen, sulfur, carbon, fluorine, or phosphorus, or a cationic element, such as chromium, iron, cobalt, or manganese, is preferably used. Further, titanium oxide fine particles in the form of a powder, or a sol or slurry obtained by dispersing the particles in an organic solvent or water can be used. Examples of commercially available titanium oxide fine particles in the form of a powder include Aerosil P-25, manufactured by Nippon Aerosil Co., Ltd., and ATM-100, manufactured by Tayca Corporation. Further, examples of commercially available titanium oxide fine particles in the form of a slurry include TKD-701, manufactured by Tayca Corporation.

The curable resin composition of the invention may further contain a fibrous substrate. With respect to the fibrous substrate, there is no particular limitation, and preferred is a fibrous substrate used in a fiber reinforced resin, and examples thereof include an inorganic fiber and an organic fiber.

Examples of inorganic fibers include inorganic fibers, such as a carbon fiber, a glass fiber, a boron fiber, an alumina fiber, and a silicon carbide fiber, a carbon fiber, an activated carbon fiber, a graphite fiber, a tungsten carbide fiber, a silicon carbide fiber, a ceramic fiber, a natural fiber, a mineral fiber, such as basalt, a boron nitride fiber, a boron carbide fiber, and a metal fiber. Examples of the metal fibers include an aluminum fiber, a copper fiber, a brass fiber, a stainless steel fiber, and a steel fiber.

Examples of organic fibers include synthetic fibers formed from a resin material, such as polybenzazole, aramid, PBO (polyparaphenylenebenzoxazole), polyphenylene sulfide, polyester, acryl, polyamide, polyolefin, polyvinyl alcohol, or polyarylate; natural fibers, such as cellulose, pulp, cotton, wool, and silk; and regenerated fibers, such as protein, polypeptide, and alginic acid.

Of these, preferred are a carbon fiber and a glass fiber, which can be industrially used in a wide range. A single type of the fiber can be used, or two or more types of the fibers can be used in combination.

The fibrous substrate may be of an aggregate form of fibers, and the fiber may be continuous or discontinuous, and the fibrous substrate may be in the form of woven fabric or nonwoven fabric. Further, the fibrous substrate may be a fiber bundle in which fibers are arranged in one direction, or in the form of a sheet having fiber bundles arranged. Further, the fibrous substrate may be in a three-dimensional form in which an aggregate form of fibers has an increased thickness.

In the curable resin composition of the invention, a dispersing medium can be used in order to adjust the solids content or viscosity of the resin composition. The dispersing medium may be any liquid medium as long as the effects of the invention are not sacrificed, and examples thereof include various organic solvents and a liquid organic polymer.

Examples of the organic solvents include ketones, such as acetone, methyl ethyl ketone (MEK), and methyl isobutyl ketone (MIBK); cyclic ethers, such as tetrahydrofuran (THF) and dioxolane; esters, such as methyl acetate, ethyl acetate, and butyl acetate; aromatic solvents, such as toluene and xylene; and alcohols, such as carbitol, Cellosolve, methanol, isopropanol, butanol, and propylene glycol monomethyl ether, and these solvents can be used alone or in combination, and especially, methyl ethyl ketone is preferred in view of the volatility during application and recovery of the solvent.

The liquid organic polymer means a liquid organic polymer which does not directly participate in the curing reaction, and examples of such organic polymers include an acrylic polymer (FLOWLEN WK-20: Kyoeisha Chemical Co., Ltd.), an amine salt of special modified phosphate (HIPLAAD ED-251: Kusumoto Chemicals Ltd.), and a modified acrylic block copolymer (DISPERBYK 2000; BYK-Chemie GmbH).

The resin composition of the invention may have another additive. Examples of additives include a catalyst, a polymerization initiator, an inorganic pigment, an organic pigment, a loading pigment, clay minerals, a wax, a surfactant, a stabilizer, a flow control agent, a coupling agent, a dye, a leveling agent, a rheology control agent, an ultraviolet light absorber, an antioxidant, a flame retardant, a plasticizer, and a reactive diluent.

A cured article can be obtained by curing the resin composition of the invention. When curing the resin composition, the resin composition may be cured at ordinary room temperature or by heating. When heat-curing is conducted, the resin composition may be cured by heating once, or may be cured through a multistage heating step.

Further, the curable resin composition of the invention can be cured by an active energy ray. In this instance, a cationic photopolymerization initiator can be used as a polymerization initiator. With respect to the active energy ray, a visible light, an ultraviolet light, an X-ray, an electron beam, or the like can be used.

Examples of cationic photopolymerization initiators include an aryl-sulfonium salt and an aryl-iodonium salt, and, specifically, an arylsulfonium hexafluorophosphate, an arylsulfonium hexafluoroantimonate, an arylsulfonium tetrakis(pentafluoro)borate salt, a tri(alkylphenyl)sulfonium hexafluorophosphate, or the like can be used. The cationic photopolymerization initiators may be used alone or two or more kinds thereof may be used in combination.

The curable resin composition of the invention may be prepared by uniformly mixing the above-mentioned components, and there is no particular limitation with respect to the method for mixing. For example, the curable resin composition can be prepared by uniformly mixing the components using a pot mill, a ball mill, a bead mill, a roll mill, a homogenizer, a super mill, a homodisper, a universal mixer, a Banbury mixer, a kneader, or the like.

The curable resin composition of the invention can be obtained by dissolving the glycidyl group-containing compound of the invention and the compound (I) having reactivity with the glycidyl group-containing compound, and further, if necessary, the above-mentioned usable curing agent, filler, fibrous substrate, and dispersing medium and a resin other than the above-mentioned compounds in a dispersing medium, such as the above-mentioned organic solvent, and then distilling off the solvent, and subjecting the resultant product to vacuum drying using a vacuum oven or the like. Further, the curable resin composition of the invention may be in such a state that the above-mentioned constituent materials are uniformly mixed. **In** this case, it is preferred that the materials are uniformly mixed using a mixer or the like. The formulation of the constituent materials can be appropriately controlled according to the desired properties of the cured article, such as mechanical strength, heat resistance, healing ability, and remoldability. Further, in the preparation of the curable resin composition, with respect to the specific order of mixing the constituent materials, there is no particular limitation.

The cured article of the invention is obtained by curing, by the glycidyl group-containing compound of the invention, the compound (I) having reactivity with the glycidyl group-containing compound. With respect to the curing method, a known method can be appropriately selected and employed according to the properties of the compound (I) having reactivity with the glycidyl group-containing compound used.

The cured article of the invention is cured by the glycidyl group-containing compound of the invention as mentioned above, and therefore the compound causes the cured article to have an appropriate crosslinking density, so that the cured article can maintain excellent mechanical strength. Further, when mechanical energy, such as a flaw or external force, is applied to the cured article of the invention, the reversible bonding is broken, but it is considered that the equilibrium shifts in the direction of bonding, and hence an adduct is formed again, enabling healing of the flaw or remolding.

The structure of the cured article obtained can be found by an infrared absorption (IR) spectrum method using Fourier transform infrared spectroscopy (FT-IR) or the like, an elemental analysis method, an X-ray scattering method, or the like.

The cured article which is an embodiment of the invention can be obtained using the glycidyl group-containing compound of the invention as one component of a curable resin composition as mentioned above, but the cured article can be obtained while forming (in situ synthesizing) the glycidyl group-containing compound using the above-mentioned conjugated diene and dienophile each having at least one glycidyl group, which are an intermediate of the glycidyl group-containing compound, during curing of the compound.

The curable resin composition of the invention and a cured article produced from the curable resin composition have dismantlability and healing ability as well as remoldability, and are advantageously used in the below-mentioned applications.

The curable resin cured article of the invention can be laminated on a substrate to form a laminated product. As a substrate for the laminated product, an inorganic material, such as a metal or glass, an organic material, such as a plastic or wood material, or the like can be appropriately used according to the use, and the substrate may be in the form of a laminated product, or in a flat plate or sheet form, or may have a three-dimensional structure, or may be in a three-dimensional form. The substrate may be in an arbitrary form according to the purpose, such as a form having curvature in the entire surface or part of the surface. Further, there is no particular limitation with respect to the hardness, thickness, and the like of the substrate. Further, a multilayer laminated product may be obtained by laminating a first substrate, a layer formed from the cured article of the curable resin composition of the invention, and a second substrate in this order. The curable resin composition of the present embodiment has excellent adhesion properties, and therefore can be advantageously used as an adhesive for bonding together the first substrate and the second substrate. Further, the cured article of the invention may be further laminated on the curable resin cured article of the invention as a substrate.

Further, the curable resin cured article of the invention can relax a stress, and therefore can be advantageously used particularly for bonding different materials. For example, even in the laminated product having different materials, such as a laminated product having a metal and/or a metal oxide as the substrate and a plastic layer as the second substrate, the adhesive force is maintained due to the stress relaxation ability of the cured article of the invention.

In the laminated product obtained by laminating the cured article of the invention and a substrate on one another, a layer comprising the cured article may be formed by directly applying or molding the composition onto the substrate, or the composition which has been molded may be laminated on the substrate. When directly applying the composition, there is no particular limitation with respect to the application method, and examples of application methods include a spraying method, a spin coating method, a dipping method, a roll coating method, a blade coating method, a doctor roll method, a doctor blade method, a curtain coating method, a slit coating method, a screen printing method, and an ink-jet method. When directly molding the composition, examples of molding methods include in-mold decorating, insert molding, vacuum forming, extrusion lamination molding, and press molding. When laminating the composition which has been molded, the uncured or semicured composition layer may be laminated and then cured, or a layer comprising the cured article obtained by completely curing the composition may be laminated on the substrate. Further, the cured article of the invention may be laminated on a substrate by applying a precursor which can form the substrate to the cured article and curing the precursor, or by bonding a precursor which can form the substrate or the composition of the invention in a state that the precursor or composition is uncured or semicured, and then curing the precursor or composition. With respect to the precursor which can form the substrate, there is no particular limitation, and examples of such precursors include various types of curable resin compositions.

The cured article obtained using the curable resin composition of the invention has especially high adhesion properties to a metal and/or a metal oxide, and therefore can be advantageously used as a primer for a metal. Examples of metals include copper, aluminum, gold, silver, iron, platinum, chromium, nickel, tin, titanium, zinc, various alloys, and composite materials thereof, and examples of metal oxides include single metal oxides and/or composite oxides of the above metals. The cured article has excellent adhesive force particularly to iron, copper, and aluminum, and therefore can be advantageously used as an adhesive for iron, copper, and aluminum.

The curable resin composition of the invention can be advantageously used as an adhesive for structural members in the fields of automobile, train, civil engineering and construction, electronics, aircraft, and aerospace industry. The adhesive can maintain such high adhesion properties without being affected by a change of the temperature of an environment that the adhesive is unlikely to suffer peeling or the like even when used for bonding different materials, for example, bonding a metal and a nonmetal. Further, the adhesive can be used as an adhesive for structural member application, an adhesive for general office application, an adhesive for medical application, an adhesive for carbon fiber, and an adhesive for a cell, module, and casing of a storage battery, and can be used as an adhesive for optical parts bonding, an adhesive for optical disc lamination, an adhesive for printed wiring board mounting, a die bonding adhesive, an adhesive for semiconductor, such as an underfill, and an adhesive for mounting, such as a BGA reinforcing underfill, an isotropic conductive film, and an isotropic conductive paste.

When the curable resin composition of the invention has a fibrous substrate and the fibrous substrate is a reinforcing fiber, the curable resin composition containing the fibrous substrate can be used as a fiber reinforced resin. With respect to the method for adding a fibrous substrate to the composition, there is no particular limitation as long as the effects of the invention are not sacrificed, and there can be mentioned a method in which the fibrous substrate and the composition are combined to form a composite using a method, such as kneading, application, impregnation, injection, or press bonding, and the method can be appropriately selected according to the form of the fiber and the use of the fiber reinforced resin.

With respect to the method for molding the fiber reinforced resin, there is no particular limitation. When producing a product in a plate form, an extrusion method is generally used, but plane pressing can be used. In addition, an extrusion method, a blow molding method, a compression molding method, a vacuum forming method, an injection molding method, and the like can be used. Further, when producing a product in a film form, a melt-extrusion method or a solution casting method can be used, and, when using a melt-molding method, examples of the methods include inflation film forming, cast molding, extrusion lamination molding, calender molding, sheet forming, fiber forming, blow molding, injection molding, rotary molding, and cover molding. Further, in the case of a resin which is cured by an active energy ray, a cured article can be produced by a curing method using an active energy ray. Particularly, when a thermosetting resin is used as a main component of the matrix resin, examples of molding methods include a molding method in which a prepreg of a molding material is formed and pressed and heated by pressing or using an autoclave, and further include RTM (Resin Transfer Molding), VaRTM (Vacuum assist Resin Transfer Molding), lamination molding, and hand lay-up molding.

With respect to the curable resin composition of the invention, a cured article obtained using the composition has healing ability, remoldability, and dismantlability, and therefore the curable resin composition can be used in a casting material for a large-size casing, a motor housing, and the inside of a casing, and a molding material for a gear, a pulley, and the like. These materials may be a cured article of a resin alone, and may be a cured article reinforced with a fiber, such as glass chips.

The fiber reinforced resin can form an uncured or semicured state called a prepreg. The cured article may be formed by circulating a product in the form of a prepreg and then finally curing the product. When forming a laminated product, it is preferred that a prepreg is formed and then another layer is laminated on the prepreg, followed by final curing, because a laminated product having the layers closely bonded together can be formed. With respect to the mass ratio of the composition and fibrous substrate used in this case, there is no particular limitation, and generally, the mass ratio is preferably controlled so that the content of the resin in the prepreg becomes 20 to 60% by mass.

The cured article of the invention has healing ability and dismantlability as well as remoldability, and especially when a combination of materials having high dissociation temperature is used, the cured article can be used as a heat-resistant material and an electronic material. Particularly, the cured article can be advantageously used in a semiconductor encapsulation material, a circuit board, a build-up film, a build-up substrate, an adhesive, and a resist material. Further, the cured article can be advantageously used in a matrix resin of a fiber reinforced resin, and is especially suitable for a prepreg having high heat resistance. The thus obtained heat-resistant members and electronic members can be advantageously used in various applications, and examples of applications include industrial mechanical parts, general mechanical parts, parts for automobile, railways, rolling stock and the like, aerospace and aircraft related parts, electronic and electric parts, building materials, container and packaging members, articles for daily use, articles for sports and leisure time amusement, and a housing member for wind power generation, but the application is not limited to these.

Especially, the cured article has such a characteristic feature that the dismantlability and the like are excellent, and thus can be advantageously used as an adhesive for structural members in the fields of automobile, train, civil engineering and construction, electronics, aircraft, and aerospace industry. The adhesive in the invention can maintain high adhesion properties even when used for bonding different materials, for example, bonding a metal and a nonmetal. Further, the adhesive in the invention can be used as an adhesive for structural member application, an adhesive for general office application, an adhesive for medical application, an adhesive for carbon fiber, and an adhesive for a cell, module, and casing of a storage battery, and examples of such adhesives include an adhesive for optical parts bonding, an adhesive for optical disc lamination, an adhesive for printed wiring board mounting, a die bonding adhesive, an adhesive for semiconductor, such as an underfill, and adhesives for mounting, such as a BGA reinforcing underfill, an isotropic conductive film, and an isotropic conductive paste.

Representative products are described with reference to the following examples.

### 1. Semiconductor encapsulation material

As a method for obtaining a semiconductor encapsulation material from the resin composition of the invention, there can be mentioned a method in which the resin composition and a curing accelerator, and an additive, such as an inorganic filler, are satisfactorily melt-mixed, if necessary, using an extruder, a kneader, a roll, or the like until the resultant mixture becomes uniform. In this instance, as an inorganic filler, fused silica is generally used, but, when the semiconductor encapsulation material is used as a high thermal conductive semiconductor encapsulation material for a power transistor or a power IC, a highly-filled crystalline silica, alumina silicon nitride or the like, which has a thermal conductivity higher than that of fused silica, or fused silica, crystalline silica, alumina, silicon nitride, or the like may be used. With respect to the filling ratio of the inorganic filler, the amount of the inorganic filler used is preferably in the range of 30 to 95% by mass, relative to 100 parts by mass of the curable resin composition, and especially for improving the flame retardancy, moisture resistance, and solder crack resistance and reducing the coefficient of linear expansion, the amount of the inorganic filler used is more preferably 70 parts by mass or more, further preferably 80 parts by mass or more.

### 2. Semiconductor device

As a semiconductor package molding method for obtaining a semiconductor device from the curable resin composition of the invention, there can be mentioned a method in which the semiconductor encapsulation material is cast, or molded using a transfer molding machine, an injection molding machine, or the like, and further heated at 50 to 250°C for 2 to 10 hours.

### 3. Printed circuit board

As a method for obtaining a printed circuit board from the composition of the invention, there can be mentioned a method in which the prepreg is laminated by a general method, and a copper foil is appropriately stacked thereon, and the resultant laminate is subjected to hot press under a pressure of 1 to 10 MPa at 170 to 300°C for 10 minutes to 3 hours.

### 4. Flexible substrate

As a method for producing a flexible substrate from the crosslinkable resin composition of the invention, there can be mentioned a method comprising the following three steps. The first step is a step in which the crosslinkable resin composition having blended a resin component, an organic solvent, and the like is applied to an electrical insulating film using a coating machine, such as a reverse-roll coater or a comma coater, the second step is a step in which the electrical insulating film having the crosslinkable resin composition applied thereto is heated using a heating apparatus at 60 to 170°C for 1 to 15 minutes to cause the solvent to volatilize from the electrical insulating film, so that the crosslinkable resin composition is B-staged, and the third step is a step in which a metal foil is hot-pressed (the pressing pressure is preferably 2 to 200 N/cm, and the pressing temperature is preferably 40 to 200°C) on the electrical insulating film having the B-staged crosslinkable resin composition as an adhesive using a heated roll or the like. When satisfactory adhesion performance can be obtained after the above-mentioned three steps, the procedure may be completed, but, when perfect adhesion performance is needed, it is preferred that the obtained flexible substrate is further subjected to post-curing under conditions at 100 to 200°C for 1 to 24 hours. The finally cured resin composition layer preferably has a thickness in the range of 5 to 100 µm.

### 5. Build-up substrate

As a method for obtaining a build-up substrate from the composition of the invention, for example, there can be mentioned a method comprising the following steps: a step in which the composition having appropriately blended a rubber, a filler, and the like is first applied to a circuit board having a circuit formed thereon using a spray coating method, a curtain coating method, or the like, and then cured (step 1); a step in which then, if necessary, perforation for a predetermined through-hole portion or the like is conducted, and then the resultant substrate is treated with a roughening agent, and the surface is washed with warm water to form an uneven surface, and subjected to plating treatment with a metal, such as copper (step 2); and a step in which the operation is repeated desired times to alternately build up a resin insulating layer and a conductor layer of a predetermined circuit pattern (step 3). Note that the perforation for a through-hole portion is conducted after forming the resin insulating layer as the outermost layer. Further, the build-up substrate in the invention can be produced by hot-pressing at 170 to 300°C a copper foil having a resin, which is obtained by semicuring the resin composition on the copper foil, on a wiring board having a circuit formed thereon, and, in this case, the step of forming a roughened surface and subjecting the surface to plating treatment can be omitted.

### 6. Build-up film

With respect to the method for obtaining a build-up film from the composition of the invention, a build-up film can be produced by applying the composition to the surface of a support film (Y) which is a substrate, and further drying the organic solvent by heating, or heated air blowing or the like to form a layer (X) of the composition.

With respect to the organic solvent used, for example, ketones, such as acetone, methyl ethyl ketone, or cyclohexanone; acetates, such as ethyl acetate, butyl acetate, Cellosolve acetate, propylene glycol monomethyl ether acetate, or carbitol acetate; carbitols, such as Cellosolve or butylcarbitol; aromatic hydrocarbons, such as toluene or xylene; dimethylformamide, dimethylacetamide, N-methylpyrrolidone, or the like are preferably used, and the organic solvent is preferably used in such an amount that the nonvolatile content of the resultant composition becomes 30 to 60% by mass.

The thickness of the formed layer (X) is generally the thickness of the conductor layer or more. The thickness of the conductor layer of the circuit board is generally in the range of 5 to 70 µm, and therefore the resin composition layer preferably has a thickness of 10 to 100 µm. The layer (X) of the composition in the invention may be protected by the below-mentioned protective film. By protecting the layer with the protective film, it is possible to prevent deposition of contaminants or the like or formation of a flaw on the surface of the resin composition layer.

Examples of the support films and protective films above-described include polyolefins, such as polyethylene, polypropylene, and polyvinyl chloride, polyesters, such as polyethylene terephthalate (hereinafter, frequently referred to simply as "PET") and polyethylene naphthalate, polycarbonate, polyimide, release paper, and metal foils, such as a copper foil and an aluminum foil. The support film and protective film may have been subjected to matte treatment, corona discharge treatment, or release treatment. With respect to the thickness of the support film, there is no particular limitation, and the support film used generally has a thickness of 10 to 150 µm, preferably in the range of 25 to 50 µm. Further, the protective film preferably has a thickness of 1 to 40 µm.

The support film (Y) is released after laminated on the circuit board, or after forming the insulating layer by heat-curing. When the support film (Y) is released after the curable resin composition layer constituting the build-up film is heat-cured, it is possible to prevent deposition of contaminants or the like in the curing step. When the support film (Y) is released after the curing, generally, the support film is preliminarily subjected to release treatment.

A multilayer printed circuit board can be produced using the build-up film obtained as mentioned above. For example, when the layer (X) is protected by a protective film, the protective film is removed, and then the layer (X) is laminated on one surface or both surfaces of a circuit board by, for example, a vacuum lamination method so as to be directly in contact with the circuit board. The lamination method may be conducted in any of a batchwise manner and a continuous manner using rolls. Further, if necessary, the build-up film and circuit board may be heated (preheated) before lamination. With respect to the conditions for lamination, the pressing temperature (lamination temperature) is preferably 70 to 140°C, the pressing pressure is preferably 1 to 11 kgf/cm² (9.8 x 10⁴ to 107.9 x 10⁴ N/m²), and lamination is preferably conducted under a reduced pressure, i.e., under an air pressure of 20 mmHg (26.7 hPa) or less.

### 7. Conductive paste

As a method for obtaining a conductive paste from the composition of the invention, for example, there can be mentioned a method in which conductive particles are dispersed in the composition. The conductive paste can be used as a paste resin composition for circuit connection or an isotropic conductive adhesive depending on the type of the conductive particles used in the paste.

### EXAMPLES

Hereinbelow, the invention will be described in more detail with reference to the following Examples and Comparative Examples, and, in the following Examples, "part(s)" and "%" are given by mass unless otherwise specified. The following Examples should not be construed as limiting the scope of the invention.

¹H- and ¹³C-NMR, FD-MS spectrum, and GPC were measured under the conditions shown below.
¹H-NMR: "JNM-ECA600", manufactured by JEOL RESONANCE Inc.
Magnetic field intensity: 600 MHz
Number of accumulation: 32
Solvent: CDCl₃, DMSO-d₆
Sample concentration: 30% by mass
¹³C-NMR: "JNM-ECA600", manufactured by JEOL RESONANCE Inc.
Magnetic field intensity: 150 MHz
Number of accumulation: 320
Solvent: DMSO-d₆
Sample concentration: 30% by mass
FD-MS: "JMS-T100GC AccuTOF", manufactured by JEOL Ltd.
Measuring range: m/z = 50.00 to 2000.00
Change rate: 25.6 mA/min
Final current: 40 mA
Cathode voltage: -10 kV
GPC: "HLC-8320GPC", manufactured by Tosoh Corp.
Column: "TSK-GEL G2000HXL" + "TSK-GEL G3000HXL" + "TSK-GEL G4000HXL", manufactured by Tosoh Corp.
Detector: RI (differential refractometer)
Conditions for measurement: 40°C
Mobile phase: Tetrahydrofuran
Flow rate: 1 ml/min
Standard: "PStQuick A", "PStQuick B", "PStQuick E", "PStQuick F", manufactured by Tosoh Corp.

With respect to the epoxy equivalent of the synthesized epoxy resin, measurement was conducted in accordance with JIS K7236 to determine an epoxy equivalent (g/eq).

As an example of a determination method for the number of the repeating units, there can be mentioned determination from the results of various appropriate instrumental analysis, such as GPC molecular weight measurement, FD-MS, or NMR.

### Synthesis Example 1

Into a flask equipped with a thermometer and a stirrer were charged 151 g (1.0 mol) of 4-hydroxyacetanilide, 300 g of acetone, and 166 g (1.2 mol) of potassium carbonate, and the temperature of the resultant mixture was increased to the reflux temperature. Then, 144 g (1.2 mol) of allyl bromide was dropwise added, and further the resultant mixture was subjected to reaction for 7 hours. After cooling to room temperature, the potassium carbonate was removed by filtration, and acetone and the excess amount of allyl bromide in the filtrate were evaporated under a reduced pressure using an evaporator. The obtained solids were dissolved in 222 g of ethanol, and 417 g (4.0 mol) of a 35% aqueous solution of hydrochloric acid was dropwise added, and the resultant mixture was subjected to reaction at 60 to 65°C for 12 hours. After cooling to room temperature, the reaction mixture was neutralized with 880 g (4.4 mol) of a 20% aqueous solution of sodium hydroxide, and extracted with 373 g of ethyl acetate three times. The resultant organic layer was dehydrated over sodium sulfate, and then ethyl acetate was evaporated under a reduced pressure using an evaporator, obtaining 113 g of 4-allyloxyaniline (0.76 mol; yield: 76%).

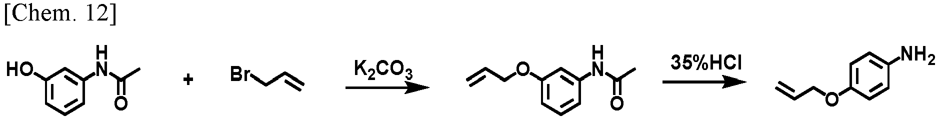

In a flask equipped with a thermometer and a stirrer, 82 g (0.84 mol) of maleic anhydride was placed, and dissolved in 1,590 g of toluene and 9.0 g of DMF. While maintaining the temperature at 10°C or lower in an ice bath, 113 g (0.76 mol) of 4-allyloxyaniline dissolved in 150 g of DMF was dropwise added to the solution, and then the resultant mixture was subjected to reaction at 25°C for 2 hours. Subsequently, 10 g of 95% sulfuric acid was added, and the resultant mixture was subjected to reaction at 110°C for 5 hours, and then toluene was distilled off under a reduced pressure. The resultant product was extracted with 1,200 g of ethyl acetate three times, and then washed with a 2% aqueous solution of sodium hydrogencarbonate twice. The resultant organic layer was dehydrated over sodium sulfate, and then ethyl acetate was evaporated under a reduced pressure using an evaporator, obtaining 150 g of N-allyloxyphenylmaleimide (0.66 mol; yield: 87%).

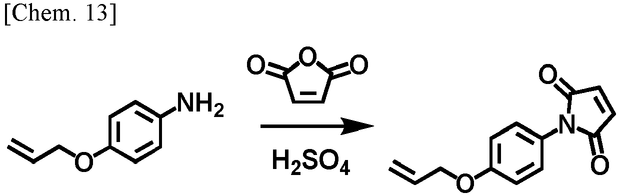

Into a flask equipped with a thermometer and a stirrer were charged 150 g (0.66 mol) of N-allyloxyphenylmaleimide and 3,390 g of dichloromethane, dissolving the compound. While maintaining the temperature at 10°C or lower in an ice bath, 1,200 g of m-chlorobenzoic acid was dropwise added, and then the resultant mixture was subjected to reaction at room temperature for 72 hours. The reaction mixture was neutralized with 402 g of a saturated aqueous solution of sodium hydrogencarbonate, and then 403 g of a saturated aqueous solution of sodium sulfite was dropwise added to the mixture. The aqueous layer was removed, and then the organic layer was washed with a 2% aqueous solution of sodium hydrogencarbonate three times and washed with water four times. The resultant organic layer was dehydrated over sodium sulfate, and then dichloromethane was evaporated under a reduced pressure using an evaporator, obtaining 60 g of a brown solid (0.24 mol; yield: 37%). The epoxy equivalent was 253 g/eq. From a ¹H-NMR analysis, it was found that 4-glycidyloxyphenylmaleimide (M-1) was obtained. ¹H-NMR (DMSO-d₆, 600 MHz): 2.72-2.87 (m, 2H), 3.84-3.90 (m, 1H), 4.36 (dd, 1H), 4.39 (dd, 1H), 7.16 (s, 2H), 7.03-7.30 (m, 4H).

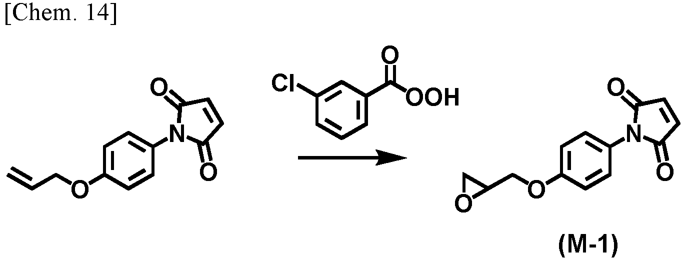

### Synthesis Example 2

In a flask equipped with a thermometer, a dropping funnel, a condenser, and a stirrer, which was placed in an ice-water bath, 180 ml of dimethylacetamide was added to 38.8 g (0.20 mol) of 9-anthrone in a nitrogen gas atmosphere to form a slurry, and 110 g (0.22 mol) of a 8.09% aqueous solution of sodium hydroxide was added to the slurry. Then, 27.7 g (0.20 mol) of epibromohydrin dissolved in 40 ml of dimethylacetamide was dropwise added to the mixture. The resultant mixture was stirred for one hour, and then the ice-water bath was removed, and 70 g of water was added, and the deposited solids were collected by filtration, and then subjected to vacuum drying, obtaining 23.6 g of a solid (yield: 47%). From a ¹H-NMR analysis, it was found that 9-(2-glycidyloxy)anthracene (A-1) was obtained. The epoxy equivalent was 265 g/eq. ¹H-NMR (CDCl₃, 600 MHz): δ = 2.79-2.96 (m, 2H), 3.54-3.62 (m, 1H), 4.17 (dd, 1H), 4.50 (dd, 1H), 7.40-7.56 (m, 4H), 7.94-8.05 (m, 2H), 8.24 (s, 1H), 8.30-8.42 (m, 2H).

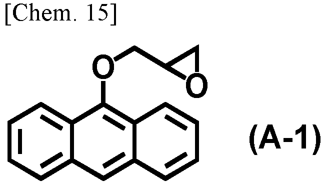

### Synthesis Example 3

Into a flask equipped with a thermometer, a dropping funnel, a condenser, and a stirrer were charged 18.8 g of 9-(4-hydroxybenzyl)-10-(4-hydroxyphenyl)anthracene (product name: BIP-ANT, manufactured by Asahi Yukizai Corporation; hydroxyl equivalent: 188 g/eq), 92.5 g (1.0 mol) of epichlorohydrin, and 23 g of n-butanol while purging the flask with nitrogen gas, dissolving the compound. The temperature of the resultant mixture was increased to 65°C, and then the pressure was reduced to such a pressure that azeotropic distillation occurred, and 7.35 g (0.09 mol) of a 49% aqueous solution of sodium hydroxide was dropwise added to the mixture over 5 hours. Then, the resultant mixture was stirred under the same conditions for 0.5 hours. During the stirring, a reaction was conducted while separating the distillate distilled by azeotropic distillation using a Dean-Stark trap, removing the aqueous layer and returning the oil layer back into the reaction system. Then, the unreacted epichlorohydrin was distilled off under a reduced pressure. To the obtained crude product, 150 g of methyl isobutyl ketone and 150 g of n-butanol were added to dissolve the product. Further, 10 g of a 10% aqueous solution of sodium hydroxide was added to the obtained solution, and the resultant mixture subjected to reaction at 80°C for 2 hours, and then the reaction mixture was washed with 50 g of water three times until the pH of the washing water had become neutral. Then, the inside of the system was dehydrated by azeotropic distillation, and the resultant product was subjected to microfiltration, and then the solvent was distilled off under a reduced pressure, obtaining 22.0 g of a glycidyl group-containing anthracene compound (A-2). The epoxy equivalent was 257 g/eq.

Further, a mass spectrum of the glycidyl group-containing anthracene compound (A-2) was measured, and a peak of M+ = 488, which corresponds to the theoretical structure represented by the structural formula below, was obtained, and, from this, it was found that the glycidyl group-containing anthracene compound (A-2) contained the structural formula below.

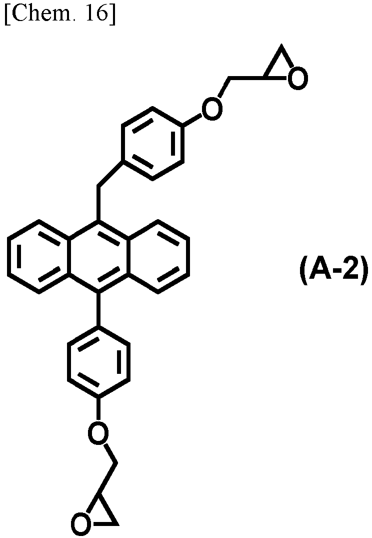

### Synthesis Example 4

The same procedure as in Synthesis Example 3 was conducted, except that 18.8 g of 9-(4-hydroxybenzyl)-10-(4-hydroxyphenyl)anthracene (product name: BIP-ANT, manufactured by Asahi Yukizai Corporation; hydroxyl equivalent: 188 g/eq) in Synthesis Example 3 was changed to 9.65 g of 2-aminoanthracene, obtaining 12.2 g of a glycidyl group-containing anthracene compound (A-3). The epoxy equivalent was 183 g/eq.

Further, a mass spectrum of the glycidyl group-containing anthracene compound (A-3) was measured, and a peak of M+ = 305, which corresponds to the theoretical structure represented by the structural formula below, was obtained, and, from this, it was found that the glycidyl group-containing anthracene compound (A-3) contained the structural formula below.

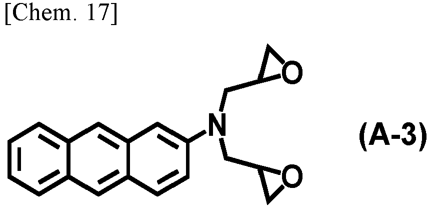

### Example 1

Into a flask equipped with a thermometer, a stirrer, and a condenser were charged 30.4 g (0.12 mol) of (M-1) obtained in Synthesis Example 1, 26.5 g (0.1 mol) of (A-1) obtained in Synthesis Example 2, 180 g of toluene, and 180 g of methyl isobutyl ketone, and the resultant mixture was subjected to reaction at 80°C for 12 hours. Then, the reaction mixture was cooled to room temperature, and the resultant precipitate was recovered by filtration by means of suction, and dried under a reduced pressure, obtaining a glycidyl group-containing compound (D-1) which is a Diels-Alder reaction adduct. The epoxy equivalent was 337 g/eq, the amount of the obtained compound was 38.9 g, and the yield was 75%.

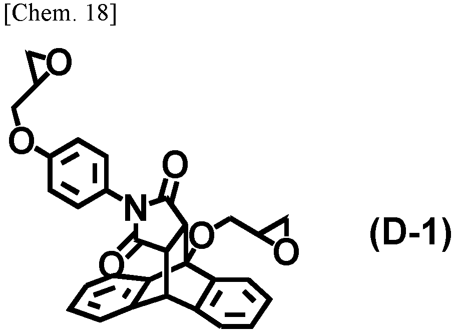

### Example 2

Into a flask equipped with a thermometer, a stirrer, and a condenser were charged 30.4 g (0.12 mol) of (M-1) obtained in Synthesis Example 1, 51.4 g (0.1 mol) of (A-2) obtained in Synthesis Example 3, 200 g of toluene, and 200 g of methyl isobutyl ketone, and the resultant mixture was subjected to reaction at 80°C for 12 hours. Then, the reaction mixture was cooled to room temperature, and the resultant precipitate was recovered by filtration by means of suction, and dried under a reduced pressure, obtaining a glycidyl group-containing compound (D-2) which is a Diels-Alder reaction adduct. The epoxy equivalent was 307 g/eq, the amount of the obtained compound was 61.4 g, and the yield was 80%.

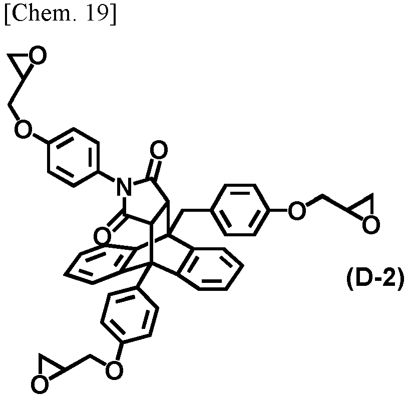

### Example 3

Into a flask equipped with a thermometer, a stirrer, and a condenser were charged 30.4 g (0.12 mol) of (M-1) obtained in Synthesis Example 1, 15.4 g (0.1 mol) of furfuryl glycidyl ether (reagent, manufactured by Merck & Co., Inc.), 180 g of toluene, and 180 g of methyl isobutyl ketone, and the resultant mixture was subjected to reaction at 80°C for 12 hours. Then, the reaction mixture was cooled to room temperature, and the resultant precipitate was recovered by filtration by means of suction, and dried under a reduced pressure, obtaining a mixture of glycidyl group-containing compounds (D-3) and (D'-3), which is a Diels-Alder reaction adduct. The epoxy equivalent was 224 g/eq, the amount of the obtained compound was 36.7 g, and the yield was 90%.

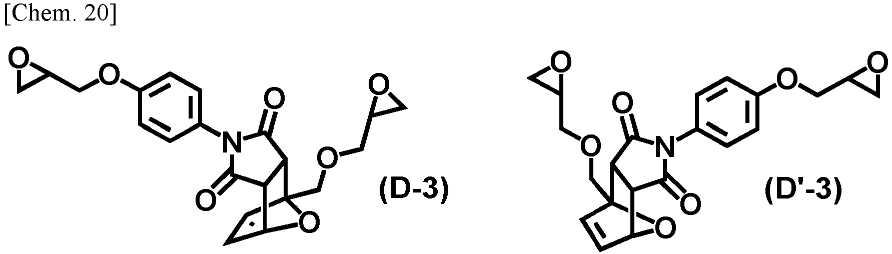

### Example 4

Into a flask equipped with a thermometer, a stirrer, and a condenser were charged 30.4 g (0.12 mol) of (M-1) obtained in Synthesis Example 1, 36.6 g (0.1 mol) of (A-4) obtained in Synthesis Example 4, 200 g of toluene, and 200 g of methyl isobutyl ketone, and the resultant mixture was subjected to reaction at 80°C for 12 hours. Then, the reaction mixture was cooled to room temperature, and the resultant precipitate was recovered by filtration by means of suction, and dried under a reduced pressure, obtaining a mixture of glycidyl group-containing compounds (D-4) and (D'-4), which is a Diels-Alder reaction adduct. The epoxy equivalent was 210 g/eq, the amount of the obtained compound was 57.0 g, and the yield was 85%.

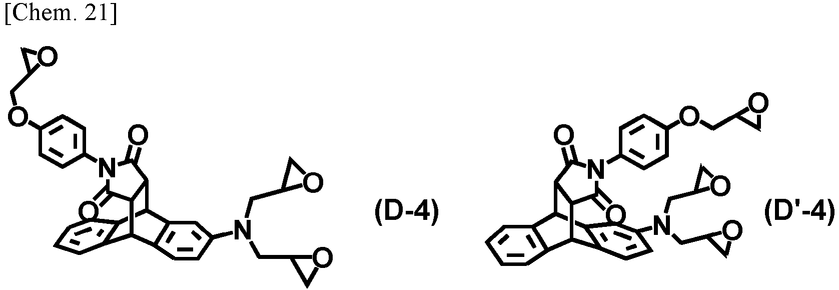

### Comparative Example 1

Into a flask equipped with a thermometer, a stirrer, and a condenser were charged 43.0 g (0.12 mol) of BMI-1000 (4,4'-diphenylmethanebismaleimide, manufactured by Daiwa Kasei Industry Co., Ltd.), 30.8 g (0.2 mol) of furfuryl glycidyl ether (reagent, manufactured by Merck & Co., Inc.), 200 g of toluene, and 200 g of methyl isobutyl ketone, and the resultant mixture was subjected to reaction at 80°C for 12 hours. Then, the reaction mixture was cooled to room temperature, and the resultant precipitate was recovered by filtration by means of suction, and dried under a reduced pressure, obtaining a Diels-Alder reaction adduct (D-5). The epoxy equivalent was 400 g/eq, the amount of the obtained compound was 40.0 g, and the yield was 60%.

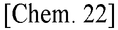

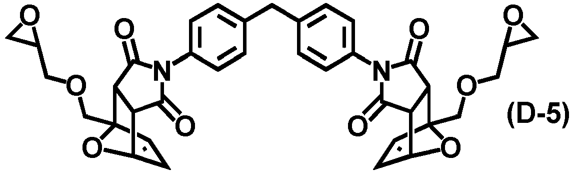

### Synthesis Example 5

In a flask equipped with a thermometer and a stirrer were placed 445 g (0.5 mol) of polytetramethylene glycol diglycidyl ether ("Denacol EX-991L", manufactured by Nagase Chemtex Corporation; epoxy equivalent: 445 g/eq) and 171 g (0.75 mol) of bisphenol A (hydroxyl equivalent: 114 g/eq), and the temperature of the resultant mixture was increased to 140°C over 30 minutes, and then 3.1 g of a 4% aqueous solution of sodium hydroxide was added. Then, the resultant mixture was increased in the temperature to 150°C over 30 minutes, and further subjected to reaction at 150°C for 16 hours. Then, sodium phosphate was added in such an amount that the reaction mixture was neutralized, obtaining 616 g of a hydroxy compound represented by the formula (Ph-1) below. In the mass spectrum of the hydroxy compound, a peak of M+ = 1,380, which corresponds to the theoretical structure of the formula below wherein m₁ = 1 and n₁ = 11, was obtained, and, from this, it was found that the hydroxy compound contained a hydroxy compound of a PTMG (polytetramethylene ether glycol) type (BPA: bisphenol A). The hydroxyl equivalent determined by GPC of the hydroxy compound (Ph-1) was 1,080 g/eq, an average of n₁ was 10.6, and an average of m₁ was 0.76.

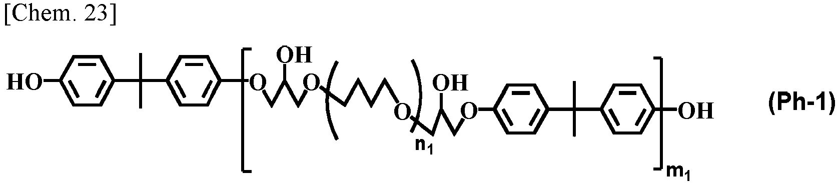

### Synthesis Example 6

In a flask equipped with a thermometer, a dropping funnel, a condenser, and a stirrer were placed 200 g of the hydroxy compound (Ph-1) obtained in Synthesis Example 5, 437 g (4.72 mol) of epichlorohydrin, and 118 g of n-butanol while purging the flask with nitrogen gas, dissolving the compound. The temperature of the resultant mixture was increased to 65°C, and then the pressure was reduced to such a pressure that azeotropic distillation occurred, and 6.66 g (0.08 mol) of a 49% aqueous solution of sodium hydroxide was dropwise added to the mixture over 5 hours.

Then, the resultant mixture was stirred under the same conditions for 0.5 hours. During the stirring, a reaction was conducted while separating the distillate distilled by azeotropic distillation using a Dean-Stark trap, removing the aqueous layer and returning the oil layer back into the reaction system. Then, the unreacted epichlorohydrin was distilled off under a reduced pressure. To the obtained crude epoxy resin, 150 g of methyl isobutyl ketone and 150 g of n-butanol were added to dissolve the resin.

Further, 10 g of a 10% aqueous solution of sodium hydroxide was added to the obtained solution, and the resultant mixture was subjected to reaction at 80°C for 2 hours, and then the reaction mixture was washed with 50 g of water three times until the pH of the washing water had become neutral.

Then, the inside of the system was dehydrated by azeotropic distillation, and the resultant product was subjected to microfiltration, and then the solvent was distilled off under a reduced pressure, obtaining 190 g of an epoxy resin (Ep-1). The epoxy equivalent of the obtained epoxy resin (Ep-1) was 1,192 g/eq. In the mass spectrum of the epoxy resin (Ep-1), a peak of M+ = 1,492, which corresponds to the theoretical structure of the formula below wherein m₁ = 1, n₁ = 11, q = 1, p₁ = 0, and p₂ = 0, was obtained, and, from this, it was found that the epoxy resin (Ep-1) contained an epoxy resin of a PTMG type (BPA).

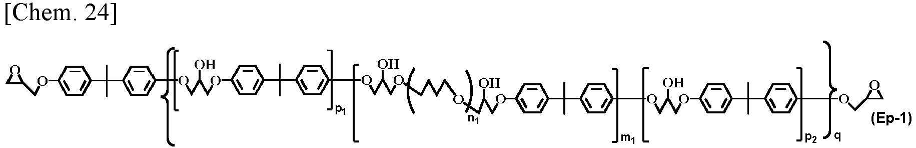

### Preparation 1 of a composition and cured article

The compounds were used in the formulation according to the Tables (the figures shown in the Tables are indicated in terms of a mass), and melt-kneaded using a two-roll mill at a temperature of 100°C for 10 minutes to obtain a curable resin composition. The obtained curable resin composition was subjected to press molding at 180°C for 60 minutes, and then further cured at 180°C for 5 hours, obtaining a cured article having a thickness of 0.7 mm.

### <Remolding test-1>

The prepared cured article was freeze-pulverized. 0.07 g of the pulverized cured article was placed in a frame, 10 mm square, having a thickness of 0.5 mm, and subjected to vacuum pressing at 150°C for 3 hours. Appearance of the obtained cured article and contamination of the mirror surface of the press plate were visually observed. The criteria are as follows.

### Appearance of the cured article

A: A seam disappeared, so that the cured article was unified.
B: A seam was visually found in part of the cured article, but the cured article was unified.
C: The cured article was in a solidified form, and broken into pieces when applying a light force.

### Contamination of the mirror surface of the press plate

A: No contaminant.
B: There was a slight contaminant deposited, which can be removed.
C: There was a contaminant deposited, which cannot be removed without using a solvent.

### <Healing test>

The prepared cured article was cut using a razor, and the resultant cut surfaces were brought into contact with each other, and then subjected to aging in a dryer at 150°C for 24 hours. The cured article was removed from the dryer, and then visually checked as to whether the cross-sections of the cured article were bonded. The criteria are as follows.
A: Bonded, and the bonded portion was not separated even when the cured article was bent 90°.
B: Bonded, and the bonded portion was separated when the cured article was bent.
C: Not bonded.

### <Dismantlability test>

The compounds were used in the formulation according to the Tables (the figures shown in the Tables are indicated in terms of a mass), and melt-kneaded using a two-roll mill at a temperature of 100°C for 10 minutes to obtain a curable resin composition. The obtained curable resin composition was uniformly spread without a gap on one of two cold-rolled steel plates ("SPCC-SD", manufactured by TP Giken Co., Ltd.; 1.0 mm x 25 mm x 100 mm), and glass beads ("J-80", manufactured by Potters-Ballotini Co., Ltd.) as a spacer were placed on the composition, and the other SPCC-SD was laminated on the spacer (bonded area: 25 mm x 12.5 mm). The resultant laminated product was heat-cured at 180°C for 60 minutes to obtain a test specimen. The test specimen was hung in a dryer at 120°C, and a load was applied to one of the substrates using a 500 g weight. The test specimen in this state was allowed to stand for 30 min, and the state of the bonded substrates was evaluated. The criteria are as follows.
A: The bonded portion was moved, and the bonded substrate to which a load was applied fell down.
B: The bonded portion was moved.
C: No change was made in the substrates.

### Preparation 2 of a composition and cured article

The compounds were blended in the formulation according to the Tables (the figures shown in the Tables are indicated in terms of a mass), and 2-ethyl-4-methyl-imidazole was added in an amount of 0.5% by mass, based on the total mass of the epoxy resin and the curing agent, and methyl ethyl ketone was added so that the nonvolatile content of the resultant composition became 58% by mass, obtaining a curable resin composition.

Then, a glass cloth (manufactured by Nitto Boseki Co., Ltd.; 2116 type; width: 210 mm x length: 280 mm x thickness: 100 µm) was impregnated with the obtained composition, and then dried at 160°C for 2 minutes to cause the solvent to volatilize, obtaining a prepreg. The six prepregs were laminated on one another and subjected to press molding at 200°C for 90 minutes, obtaining a cured article in a laminate form.

### <Remolding test-2>

The prepared flat cured article in a laminate form was placed in a mold having in the center portion a cavity having a width of 100 mm, a length of 100 mm, and a depth of 10 mm, and subjected to vacuum pressing at 180°C for 3 hours. Appearance of the obtained cured article was visually observed. The criteria are as follows.

### Appearance of the cured article

A: The obtained cured article had no crack or the like, and had been molded as same as the mold.
B: A crack was visually found in part of the obtained cured article, but the cured article had been molded as same as the mold.
C: The cured article was not able to follow deformation, causing a crack.

**[Table 1]**

| | | Example 5 | Example 6 | Example 7 | Example 8 | Example 9 | Example 10 |
|---|---|---|---|---|---|---|---|
| Epoxy resin | D-1 | 94.1 | | | | 11.4 | |
| | D-2 | | 93.6 | | | | 31.9 |
| | D-3 | | | 91.4 | | | |
| | D-4 | | | | 90.9 | | |
| | D-5 | | | | | | |
| | Ep-1 | | | | | 56.2 | 64.8 |
| | EPICLON 850S | | | | | 27.6 | |
| Curing agent | DICY | 5.9 | 6.4 | 8.6 | 9.1 | 4.8 | 3.3 |
| Accelerator | DCMU | 0.85 | 0.85 | 0.85 | 0.85 | 0.85 | 0.85 |
| DA Concentration* | mmol/g | 1.3 | 1.5 | 2 | 1.4 | 0.17 | 0.52 |
| Remolding test-1 | Appearance | A | A | A | A | A | A |
| | Contamination | A | A | B | A | A | A |
| Healing test | | A | A | A | A | A | A |
| Dismantlability test | | B | B | A | B | B | B |

| | | | | | | | |
|---|---|---|---|---|---|---|---|
| * Concentration of the Diels-Alder reaction unit, based on the total mass of the curable components of the curable resin composition | | | | | | | |

**[Table 2]**

| | | Comparative Example 2 | Comparative Example 3 | Comparative Example 4 |
|---|---|---|---|---|
| Epoxy resin | D-1 | | | |
| | D-2 | | | |
| | D-3 | | | |
| | D-4 | | | |
| | D-5 | 95 | 11.4 | |
| | Ep-1 | | 56.2 | 63.8 |
| | EPICLON 850S | | 27.6 | 31.5 |
| Curing agent | DICY | 5 | 4.8 | 4.7 |
| Accelerator | DCMU | 0.85 | 0.85 | 0.85 |
| DA Concentration* | mmol/g | 1.2 | 0.14 | 0 |
| Remolding test-1 | Appearance | B | B | C |
| | Contamination | C | C | A |
| Healing test | | B | B | C |
| Dismantlability test | | B | C | C |

EPICLON 850-S: BPA Liquid epoxy resin; epoxy equivalent: 188 g/eq
DICY: Dicyandiamide
DCMU: 3-(3,4-Dichlorophenyl)-1,1-dimethylurea

**[Table 3]**

| | | Example 11 | Example 12 | Example 13 | Example 14 | Example 15 | Example 16 |
|---|---|---|---|---|---|---|---|
| Epoxy resin | D-1 | 76.4 | | | | 9.6 | |
| | D-2 | | 74.7 | | | | 28.2 |
| | D-3 | | | 68.3 | | | |
| | D-4 | | | | 66.9 | | |
| | D-5 | | | | | | |
| | Ep-1 | | | | | 47.2 | 57.3 |
| | EPICLON 850S | | | | | 23.2 | |
| Curing agent | TD2131 | 23.6 | 25.3 | 31.7 | 33.1 | 20 | 14.5 |
| Accelerator | 2E4MZ | 0.5 | 0.5 | 0.5 | 0.5 | 0.5 | 0.5 |
| DA Concentration* | mmol/g | 1.1 | 1.2 | 1.5 | 1.1 | 0.14 | 0.46 |
| Remolding test-2 | Appearance | B | B | A | B | A | A |
| | Contamination | A | A | A | A | A | A |

**[Table 4]**

| | | Comparative Example 5 | Comparative Example 6 | Comparative Example 7 |
|---|---|---|---|---|
| Epoxy resin | D-1 | | | |
| | D-2 | | | |
| | D-3 | | | |
| | D-4 | | | |
| | D-5 | 79.4 | 9.6 | |
| | Ep-1 | | 47.2 | 53.8 |
| | EPICLON 850S | | 23.2 | 26.6 |
| Curing agent | TD2131 | 20.6 | 20 | 19.6 |
| Accelerator | 2E4MZ | 0.5 | 0.5 | 0.5 |
| DA Concentration* | mmol/g | 1 | 0.12 | 0 |
| Remolding test-2 | Appearance | B | B | C |
| | Contamination | C | C | A |

TD2131: Phenolic novolak resin; hydroxyl equivalent: 104 g/eq
2E4MZ: 2-Ethyl-4-methyl-imidazole

## Claims

1. A glycidyl group-containing compound comprising a conjugated diene structure (A) having at least one glycidyl group and a dienophile structure (B) having at least one glycidyl group, wherein the conjugated diene structure (A) and the dienophile structure (B) are linked together by the Diels-Alder reaction.

2. The glycidyl group-containing compound according to claim 1, wherein the dissociation temperature of the linked portion by the Diels-Alder reaction is 100°C or higher.

3. The glycidyl group-containing compound according to claim 1, wherein the conjugated diene structure (A) is a furan-derived structure or an anthracene-derived structure.

4. The glycidyl group-containing compound according to claim 1, wherein the dienophile structure (B) is a maleimide-derived structure.

5. A glycidyl group-containing compound which is represented by the following general formula: 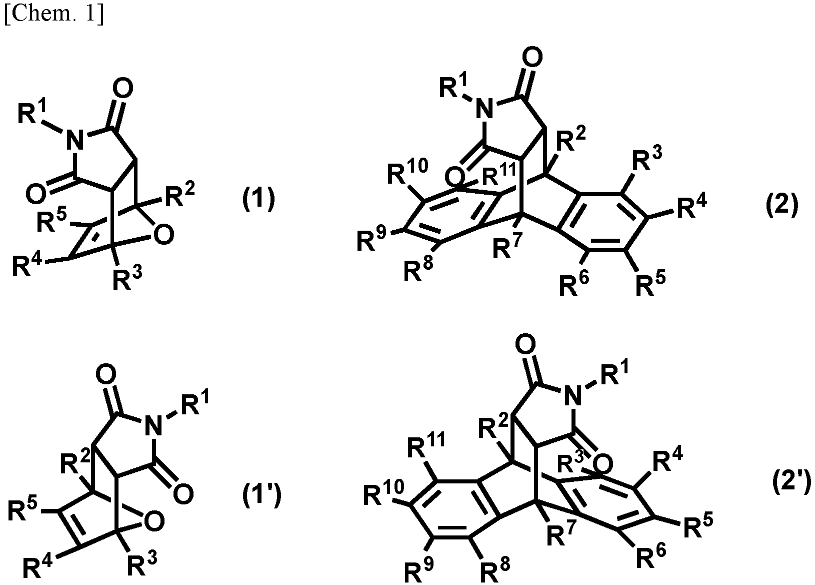 wherein R¹ is a glycidyl group or a substituent having at least one glycidyl group, and each of R² to R¹¹ is independently a hydrogen atom, a halogen atom, a hydroxyl group, a nitro group, a carboxyl group, a cyano group, an alkoxy group, an aralkyloxy group, an aryloxy group, an amino group, an amide group, an alkyloxycarbonyl group, an aryloxycarbonyl group, an alkyl group, a cycloalkyl group, an aralkyl group, an aryl group, or a glycidyl group,
and may have a substituent on the carbon atom or nitrogen atom of the alkoxy group, aralkyloxy group, aryloxy group, amino group, amide group, alkyloxycarbonyl group, aryloxycarbonyl group, alkyl group, cycloalkyl group, aralkyl group, aryl group, or glycidyl group,
with the proviso that at least one of R² to R⁵ in the formula (1) or (1') or at least one of R² to R¹¹ in the formula (2) or (2') is a glycidyl group or a group having at least one glycidyl group.

6. The glycidyl group-containing compound according to any one of claims 1 to 5, which has an epoxy equivalent in the range of 100 to 500 g/eq.

7. A curable resin composition comprising the glycidyl group-containing compound according to any one of claims 1 to 5, and a compound (I) having reactivity with the glycidyl group-containing compound as essential components.

8. The curable resin composition according to claim 7, wherein the compound (I) having reactivity with the glycidyl group-containing compound is a hydroxyl group-containing compound.

9. The curable resin composition according to claim 7, further comprising an epoxy resin which is other than the glycidyl group-containing compound according to any one of claims 1 to 5, and which has an epoxy equivalent of 100 to 10,000 g/eq.

10. The curable resin composition according to claim 9, wherein the epoxy resin is represented by the following formula (3) and has an epoxy equivalent of 500 to 10,000 g/eq: 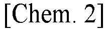 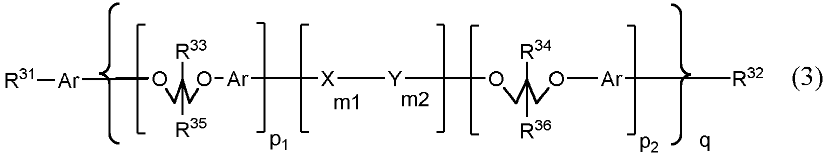
wherein, in the formula (3), each Ar is independently a structure having an aromatic ring which is unsubstituted or which has a substituent,
X is a structural unit represented by the following formula (3-1), Y is a structural unit represented by the following formula (3-2): 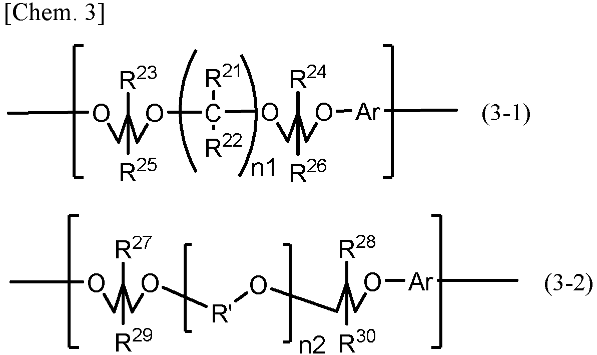
wherein, in the formulae (3-1) and (3-2), Ar is as defined above,
each of R²¹ and R²² is independently a hydrogen atom, a methyl group, or an ethyl group,
R' is a divalent hydrocarbon group having 2 to 12 carbon atoms,
each of R²³, R²⁴, R²⁷, and R²⁸ is independently a hydroxyl group, a glycidyl ether group, or a 2-methylglycidyl ether group,
each of R²⁵, R²⁶, R²⁹, and R³⁰ is independently a hydrogen atom or a methyl group,
n₁ is an integer of 4 to 16, and
n₂ represents an average of the numbers of the repeating units and is 2 to 30,
each of R³¹ and R³² is independently a glycidyl ether group or a 2-methylglycidyl ether group,
each of R³³ and R³⁴ is independently a hydroxyl group, a glycidyl ether group, or a 2-methylglycidyl ether group,
R³⁵ and R³⁶ are a hydrogen atom or a methyl group, and
m1, m2, p1, p2, and q are an average of the numbers of repetition,
wherein each of m1 and m2 is independently 0 to 25, and m1 and m2 satisfy the relationship: m₁ + m2 ≥ 1,
each of p1 and p2 is independently 0 to 5, and
q is 0.5 to 5,
with the proviso that the bonding of the structural unit X represented by the formula (3-1) above and the structural unit Y represented by the formula (3-2) above may be in a random or block form, and the total number of structural units X and the total number of structural units Y present per molecule are m1 and m2, respectively.

11. The curable resin composition according to claim 7, wherein the concentration of the Diels-Alder reaction unit is 0.10 mmol/g or more, based on the total mass of the curable components of the curable resin composition.

12. The curable resin composition according to claim 7, which is a self-heating composition, a composition for a remolding material, or a dismantlable composition.

13. A cured article which is obtained by curing the curable resin composition according to claim 7.

14. A laminated product having a substrate, and a layer comprising the cured article according to claim 13.

15. A heat-resistant member comprising the cured article according to claim 13.
